(19) Europäisches Patentamt
European Patent Office
Office européen des brevets

(11) **EP 4 506 364 A1**

(12) **EUROPEAN PATENT APPLICATION**
published in accordance with Art. 153(4) EPC

(43) Date of publication:
**12.02.2025 Bulletin 2025/07**

(21) Application number: **23778475.6**

(22) Date of filing: **31.03.2023**

(51) International Patent Classification (IPC):
**C07K 16/28** (2006.01)     **A61K 39/395** (2006.01)
**A61K 39/00** (2006.01)     **A61P 35/00** (2006.01)

(52) Cooperative Patent Classification (CPC):
**A61K 39/00; A61K 39/395; A61P 35/00;
C07K 16/28**

(86) International application number:
**PCT/CN2023/085538**

(87) International publication number:
**WO 2023/186111 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **02.04.2022 CN 202210351188**

(71) Applicant: **Harbour Biomed (Shanghai) Co., Ltd
Shanghai 201203 (CN)**

(72) Inventors:
• **LUO, Haishan
  Shanghai 201203 (CN)**
• **LI, He
  Shanghai 201203 (CN)**

• **DENG, Gang
  Shanghai 201203 (CN)**
• **WANG, Yongqiang
  Shanghai 201203 (CN)**
• **WANG, Yuandong
  Shanghai 201203 (CN)**
• **CHEN, Fei
  Shanghai 201203 (CN)**
• **RONG, Yiping
  Shanghai 201203 (CN)**

(74) Representative: **Murgitroyd & Company
Murgitroyd House
165-169 Scotland Street
Glasgow G5 8PL (GB)**

Remarks:
The complete document including Reference
Table(s) and the Sequence Listing(s) can be
downloaded from the EPO website

(54) **ANTIGEN BINDING PROTEIN TARGETING CD40, AND PREPARATION THEREFOR AND USE THEREOF**

(57)     Disclosed in the present invention are an antigen binding protein targeting CD40, and the preparation therefor and the use thereof. The antigen binding protein targeting CD40 has the characteristics of a high affinity to CD40 and a strong agonistic activity on signaling pathways, particularly the enhanced agonistic activity after crosslinking, so that the antigen binding protein targeting CD40 has a larger therapeutic window, and is expected to bring about new opportunities for the treatment of various tumors.

FIG. 1A

**Description**

The present application claims the right of priority for the Chinese patent application No. 2022103511889 with the filling date of April 2, 2022. This Chinese patent application is incorporated herein by reference in its entirety.

TECHNICAL FIELD

The present invention relates to the field of biopharmaceuticals, and in particular to an antigen binding protein targeting CD40, and the preparation therefor and the use thereof.

BACKGROUND ART

CD40 is a glycosylated type I transmembrane protein and a member of the tumor necrosis factor receptor superfamily (TNFRSF), and also known as tumor necrosis factor receptor superfamily member 5 (TNFRSF5). CD40 is expressed on the surface of a series of antigen-presenting cells (APCs), including a monocyte, a dendritic cell (DC), a B cell, and a macrophage. CD40L, a ligand of CD40, is mainly expressed on the surface of lymphocytes including a T cell, a B cell, and a natural killer cell (NK), and usually exists in the form of a trimer and a polymer. CD40 and CD40L are a pair of co-stimulatory molecules. Activation of CD40 downstream signaling pathway requires the crosslinking of CD40 to CD40L in the trimer and polymer form. CD40 and CD40L interact on the cell surface, causing CD40 to redistribute to membrane lipid rafts and undergo conformational changes. CD40 recruits TNFR-associated factor (TRAF) in the cytoplasm by the intracellular terminal domain to promote intracellular signal transduction, thereby activating different signaling pathways, such as classical and non-classical nuclear factor κB pathways, p38 mitogen-activated protein kinase, phosphatidylinositol-3 kinase (PI3K) and phospholipase Cγ pathways, and further regulating apoptosis, cell cycle progression, cytokine production and the expression of cell surface immune regulator via the genes targeted by these signaling pathways. Therefore, activation of CD40 can increase antigen presentation, promote cytokine secretion, activate lymphocyte, and simultaneously stimulate and activate the human innate immune system and acquired immune system, thereby producing a synergistic effect to resist the occurrence and development of cancers.

CD40 is also widely expressed in tumor cells and is expressed in almost all B-cell malignancies and a wide range of solid tumors, including melanoma, lung cancer, breast cancer, colon cancer, prostate cancer, pancreatic cancer, kidney cancer, ovarian cancer, and head and neck cancer. CD40 expressed on the surface of tumor cells can mediate tumor cell death. In the absence of immune helper cells, CD40 expressed on the surface of a variety of tumor cells will mediate direct cytotoxic effects by crosslinking to CD40L. In vitro, crosslinking of CD40 to CD40L has been shown to induce tumor cell apoptosis and inhibit the growth of solid tumor cells and B malignant tumor cells. In vivo, activation of CD40 also mediates inhibitory effects on tumors. Evidence indicated that in immunodeficient mice, intervention with CD40 on the surface of tumor cells by CD40L could inhibit the growth of breast cancer cell transplant tumor or B lymphocyte transplant tumor without lymphocyte activation.

Therefore, the mechanism of CD40-mediated tumor cell death can be dual, namely, stimulation of the immune system to kill tumor cells and direct tumor cytotoxicity, which can be synergistic in anti-tumor effects. An agonistic anti-CD40 antibody, similar to CD40L, can crosslink and activate CD40 on the surface of immune cells and tumor cells, exerting a significant anti-tumor effect. This anti-tumor effect has been demonstrated in trials in preclinical animal models and clinical tumor patients. The anti-CD40 antibody can be combined with chemotherapeutic drugs, such as gemcitabine and paclitaxel, or with immunomodulatory drugs, such as a PD-1 antibody and a CTLA-4 antibody, to produce a synergistic anti-tumor effect.

At present, there are 20 CD40 antibody drugs in the clinical trial stage, but the earliest product is only in Phase II clinical trials, and there is no commercially available product yet. Therefore, the development of other CD40 antibodies will provide the possibility for the treatment of various tumors and immune system-related diseases and has a high market value.

In the field of anti-tumor, the main problems of the CD40 antibody in clinical practice include low objective response rate, significant toxic side effects and low tolerated dose. These problems may be due to the weak activity of agonists, such as Celldex product CDX-1140. In Phase I clinical trial of CDX-1140, no complete or partial responses were observed in 42 patients treated with monotherapy. In the combined treatment with rhFLT3L, only 1 of the 20 patients experienced partial response. This product has a weak activation effect on DC cells in vitro, and the agonist activity cannot be enhanced in the presence of crosslinking. In clinical trials, Roche CD40 antibody, Selicrelumab, caused significant toxic side effects such as cytokine release syndrome and liver toxicity in some patients at a dosage exceeded 0.2 mg/kg. In clinical trials, Apexigen CD40 antibody, APX005M, caused significant neutropenia, as well as further sepsis and septic shock in some patients at a dosage exceeded 0.3 mg/kg. In addition, a CD40 monoclonal antibody with a human-mouse chimeric sequence, ChiLob7/4, failed in a phase I clinical trial due to the human anti-chimeric antibody (HACA). In summary, existing clinical CD40 antibodies face the problem and challenge of a too-small therapeutic window between effectiveness and safety.

SUMMARY OF THE INVENTION

In view of the technical defect of a small therapeutic window between effectiveness and safety of CD40 antibodies in the prior art, the present invention provides an antigen binding protein targeting CD40, and the preparation therefor and the use thereof. The antigen binding protein targeting CD40 has the characteristics of a high affinity to CD40 and a strong agonistic activity on signaling pathways, particularly the enhanced agonistic activity after crosslinking, so that the antigen binding protein targeting CD40 has a larger therapeutic window and good safety, and is expected to bring about new opportunities for the treatment of various tumors.

In order to solve the above technical problem, one technical solution provided in the present invention is: an antigen binding protein targeting CD40, which comprises a light chain variable region (VL) comprising LCDR1, LCDR2, and LCDR3 and a heavy chain variable region (VH) comprises HCDR1, HCDR2, and HCDR3; wherein the LCDR1 comprises an amino acid sequence set forth in SEQ ID NO: 33 or a variant 1 with 3, 2 or 1 amino acid mutation in SEQ ID NO: 33, the LCDR2 comprises an amino acid sequence set forth in SEQ ID NO: 41 or a variant 2 with 3, 2 or 1 amino acid mutation in SEQ ID NO: 41, the LCDR3 comprises an amino acid sequence set forth in SEQ ID NO: 49 or a variant 3 with 3, 2 or 1 amino acid mutation in SEQ ID NO: 49, the HCDR1 comprises an amino acid sequence set forth in SEQ ID NO: 7 or a variant 4 with 3, 2 or 1 amino acid mutation in SEQ ID NO: 7, the HCDR2 comprises an amino acid sequence set forth in SEQ ID NO: 15 or a variant 5 with 3, 2 or 1 amino acid mutation in SEQ ID NO: 15, and the HCDR3 comprises an amino acid sequence set forth in SEQ ID NO: 23 or a variant 6 with 3, 2 or 1 amino acid mutation in SEQ ID NO: 23.

Preferably, the variant 3 comprises an amino acid sequence having a PTM site mutation in the amino acid sequence set forth in SEQ ID NO: 49, preferably comprises an amino acid sequence having an amino acid mutation at position 4 and/or position 5 of the amino acid sequence set forth in SEQ ID NO: 49, the amino acid mutation is preferably an amino acid substitution, and more preferably a conservative amino acid substitution.

In some technical solutions of the present invention, the variant 3 is an amino acid sequence set forth in any one of SEQ ID NOs: 50-73.

In some technical solutions of the present invention, the LCDR1 comprises an amino acid sequence set forth in SEQ ID NO: 33, the LCDR2 comprises an amino acid sequence set forth in SEQ ID NO: 41, the LCDR3 comprises an amino acid sequence set forth in any one of SEQ ID NOs: 49-73, the HCDR1 comprises an amino acid sequence set forth in SEQ ID NO: 7, the HCDR2 comprises an amino acid sequence set forth in SEQ ID NO: 15, and the HCDR3 comprises an amino acid sequence set forth in SEQ ID NO: 23. See Table 1-1 below for specific CDR combinations.

Table 1-1: Specific CDR combinations

| | SEQ ID NO: | | | | | |
|---|---|---|---|---|---|---|
| Antibody number | LCDR1 | LCDR2 | LCDR3 | HCDR1 | HCDR2 | HCDR3 |
| PR003379 | 33 | 41 | 49 | 7 | 15 | 23 |
| PR006239 | 33 | 41 | 49 | 7 | 15 | 23 |
| PR006492 | 33 | 41 | 50 | 7 | 15 | 23 |
| PR006493 | 33 | 41 | 51 | 7 | 15 | 23 |
| PR006494 | 33 | 41 | 52 | 7 | 15 | 23 |
| PR006495 | 33 | 41 | 53 | 7 | 15 | 23 |
| PR006496 | 33 | 41 | 54 | 7 | 15 | 23 |
| PR006497 | 33 | 41 | 55 | 7 | 15 | 23 |
| PR006498 | 33 | 41 | 56 | 7 | 15 | 23 |
| PR006499 | 33 | 41 | 57 | 7 | 15 | 23 |
| PR006500 | 33 | 41 | 58 | 7 | 15 | 23 |
| PR006501 | 33 | 41 | 59 | 7 | 15 | 23 |
| PR006502 | 33 | 41 | 60 | 7 | 15 | 23 |
| PR006503 | 33 | 41 | 61 | 7 | 15 | 23 |
| PR006504 | 33 | 41 | 62 | 7 | 15 | 23 |
| PR006505 | 33 | 41 | 63 | 7 | 15 | 23 |

(continued)

| Antibody number | SEQ ID NO: | | | | | |
| --- | --- | --- | --- | --- | --- | --- |
| | LCDR1 | LCDR2 | LCDR3 | HCDR1 | HCDR2 | HCDR3 |
| PR006506 | 33 | 41 | 64 | 7 | 15 | 23 |
| PR006507 | 33 | 41 | 65 | 7 | 15 | 23 |
| PR006509 | 33 | 41 | 66 | 7 | 15 | 23 |
| PR006510 | 33 | 41 | 67 | 7 | 15 | 23 |
| PR006511 | 33 | 41 | 68 | 7 | 15 | 23 |
| PR006512 | 33 | 41 | 69 | 7 | 15 | 23 |
| PR006513 | 33 | 41 | 70 | 7 | 15 | 23 |
| PR006514 | 33 | 41 | 71 | 7 | 15 | 23 |
| PR006515 | 33 | 41 | 72 | 7 | 15 | 23 |
| PR006516 | 33 | 41 | 73 | 7 | 15 | 23 |

Preferably, the light chain variable region comprises an amino acid sequence set forth in SEQ ID NO: 85 or an amino acid sequence having at least 85%, 90%, 92%, 94%, 95%, 96%, 97%, 98% or 99% identity to SEQ ID NO: 85, and the heavy chain variable region comprises an amino acid sequence set forth in SEQ ID NO: 81 or an amino acid sequence having at least 85%, 90%, 92%, 94%, 95%, 96%, 97%, 98% or 99% identity to SEQ ID NO: 81. More preferably, the light chain variable region comprises an amino acid sequence set forth in any one of SEQ ID NOs: 85-109, and the heavy chain variable region comprises an amino acid sequence set forth in SEQ ID NO: 81. See Table 1-2 for specific combinations of light chain variable region and heavy chain variable region.

Table 1-2: Specific combinations of light chain variable region and heavy chain variable region

| Antibody number | SEQ ID NO: | |
| --- | --- | --- |
| | Light chain variable region | Heavy chain variable region |
| PR003379 | 85 | 81 |
| PR006239 | 85 | 81 |
| PR006492 | 86 | 81 |
| PR006493 | 87 | 81 |
| PR006494 | 88 | 81 |
| PR006495 | 89 | 81 |
| PR006496 | 90 | 81 |
| PR006497 | 91 | 81 |
| PR006498 | 92 | 81 |
| PR006499 | 93 | 81 |
| PR006500 | 94 | 81 |
| PR006501 | 95 | 81 |
| PR006502 | 96 | 81 |
| PR006503 | 97 | 81 |
| PR006504 | 98 | 81 |
| PR006505 | 99 | 81 |
| PR006506 | 100 | 81 |
| PR006507 | 101 | 81 |
| PR006509 | 102 | 81 |

(continued)

| | SEQ ID NO: | |
|---|---|---|
| Antibody number | Light chain variable region | Heavy chain variable region |
| PR006510 | 103 | 81 |
| PR006511 | 104 | 81 |
| PR006512 | 105 | 81 |
| PR006513 | 106 | 81 |
| PR006514 | 107 | 81 |
| PR006515 | 108 | 81 |
| PR006516 | 109 | 81 |

Preferably, the antigen binding protein targeting CD40 satisfies at least one of the following three conditions:

(1) the antigen binding protein targeting CD40 is a full-length antibody, Fab, Fab', F(ab')2, or Fv, and preferably the Fv is scFv;
(2) the antigen binding protein targeting CD40 is a monospecific antibody, a bispecific antibody, or a multispecific antibody;
(3) the antigen binding protein targeting CD40 is a monoclonal antibody or a polyclonal antibody.

Preferably, the antigen binding protein targeting CD40 is a full-length antibody comprising a light chain and a heavy chain, the light chain comprises a light chain constant region (CL), the light chain constant region is preferably a light chain constant region of human antibody, and the heavy chain comprises a heavy chain constant region (CH), and the heavy chain constant region is preferably a heavy chain constant region of human antibody, more preferably a heavy chain constant region of hIgG1, hIgG2, hIgG3 or hIgG4 subtype, further preferably a heavy chain constant region of hIgG1 subtype.

In some technical solutions of the present invention, the antigen binding protein targeting CD40 is a full-length antibody comprising a light chain and a heavy chain, the heavy chain comprises an amino acid sequence set forth in SEQ ID NO: 114 or 115, and the light chain comprises an amino acid sequence set forth in any one of SEQ ID NOs: 119-143. Preferably, the heavy chain comprises an amino acid sequence set forth in SEQ ID NO: 114, and the light chain comprises an amino acid sequence set forth in SEQ ID NO: 119, or, the heavy chain comprises an amino acid sequence set forth in SEQ ID NO: 115, and the light chain comprises an amino acid sequence set forth in any one of SEQ ID NOs: 119-143. See Table 1-3 below for specific combinations of light chain and heavy chain.

Table 1-3: Specific combinations of light chain and heavy chain

| | SEQ ID NO: | |
|---|---|---|
| Antibody number | Light chain | Heavy chain |
| PR003379 | 119 | 114 |
| PR006239 | 119 | 115 |
| PR006492 | 120 | 115 |
| PR006493 | 121 | 115 |
| PR006494 | 122 | 115 |
| PR006495 | 123 | 115 |
| PR006496 | 124 | 115 |
| PR006497 | 125 | 115 |
| PR006498 | 126 | 115 |
| PR006499 | 127 | 115 |
| PR006500 | 128 | 115 |
| PR006501 | 129 | 115 |

(continued)

|  | SEQ ID NO: | |
| --- | --- | --- |
| Antibody number | Light chain | Heavy chain |
| PR006502 | 130 | 115 |
| PR006503 | 131 | 115 |
| PR006504 | 132 | 115 |
| PR006505 | 133 | 115 |
| PR006506 | 134 | 115 |
| PR006507 | 135 | 115 |
| PR006509 | 136 | 115 |
| PR006510 | 137 | 115 |
| PR006511 | 138 | 115 |
| PR006512 | 139 | 115 |
| PR006513 | 140 | 115 |
| PR006514 | 141 | 115 |
| PR006515 | 142 | 115 |
| PR006516 | 143 | 115 |

In the present invention, the numbers in the variant 1, variant 2, variant 3, variant 4, variant 5 and variant 6 are only used to distinguish different variants and do not indicate the actual order they represent.

In the present invention, the PTM site is a site where a chemical modification is sometimes introduced after the translation synthesis of amino acid chains of proteins or polypeptides in cells, and called post-translational modification (PTM) site.

The amino acid sequence of the CDR in the present invention is determined according to rules of Chothia definition.

In order to solve the above technical problem, another technical solution provided in the present invention is: a chimeric antigen receptor (CAR) comprising the antigen binding protein targeting CD40 of the present invention.

In order to solve the above technical problem, another technical solution provided in the present invention is: an isolated nucleic acid encoding the antigen binding protein targeting CD40 of the present invention or the chimeric antigen receptor of the present invention.

In order to solve the above technical problem, another technical solution provided in the present invention is: a recombinant expression vector comprising the isolated nucleic acid of the present invention. Preferably, the recombinant expression vector comprises a eukaryotic cell expression vector and/or a prokaryotic cell expression vector.

In order to solve the above technical problem, another technical solution provided in the present invention is: a transformant comprising the isolated nucleic acid of the present invention or the recombinant expression vector of the present invention. Preferably, a host cell of the transformant is a prokaryotic cell and/or a eukaryotic cell, the prokaryotic cell is preferably an *E. coli* cell such as a TG1 or a BL21, and the eukaryotic cell is preferably an HEK293 cell or a CHO cell.

In order to solve the above technical problem, another technical solution provided in the present invention is: a method for preparing the antigen binding protein targeting CD40 of the present invention, the method comprising culturing the transformant of the present invention, and obtaining the antigen binding protein targeting CD40 from the culture.

In order to solve the above technical problem, another technical solution provided in the present invention is: an antibody-drug conjugate (ADC) comprising the antigen binding protein targeting CD40 of the present invention, and a cytotoxic agent or a label. Preferably, the cytotoxic agent is MMAF or MMAE, and the label is a fluorescent agent.

In order to solve the above technical problem, another technical solution provided in the present invention is: a genetically modified cell expressing the chimeric antigen receptor of the present invention. Preferably, the genetically modified cell is a eukaryotic cell, preferably an isolated human cell, more preferably an immune cell such as T cell or NK cell.

In order to solve the above technical problem, another technical solution provided in the present invention is: a pharmaceutical composition comprising the antigen binding protein targeting CD40 of the present invention, the isolated nucleic acid of the present invention, the recombinant expression vector of the present invention, the genetically modified cell of the present invention and/or the antibody-drug conjugate of the present invention, and a pharmaceutically acceptable carrier and/or a pharmaceutically acceptable adjuvant. Preferably, the pharmaceutical composition further comprises other anti-tumor antibodies as active ingredients.

In order to solve the above technical problem, another technical solution provided in the present invention is: a detection reagent comprising the antigen binding protein targeting CD40 of the present invention and/or the antibody-drug conjugate of the present invention. Preferably, the detection reagent is in a liquid dosage form, a gaseous dosage form, a solid dosage form and a semi-solid dosage form. More preferably, the detection reagent further comprises a secondary antibody, CD40 or a derivative thereof, the secondary antibody is, for example, an anti-human IgG antibody conjugated to horseradish peroxidase and an anti-human IgG antibody conjugated to biotin.

In order to solve the above technical problem, another technical solution provided in the present invention is: a combined kits comprising kit A containing one or more of the antigen binding protein targeting CD40 of the present invention, the pharmaceutical composition of the present invention, the detection reagent of the present invention, the genetically modified cell of the present invention and the antibody-drug conjugate of the present invention.

Preferably, the combined kits further comprises kit B containing other anti-tumor antibodies or a pharmaceutical composition comprising the other anti-tumor antibodies, and/or one or more selected from the group consisting of a hormone preparation, a targeting small molecule preparation, a proteasome inhibitor, an imaging agent, a diagnostic agent, a chemotherapeutic agent, an oncolytic drug, a cytotoxic agent, a cytokine, an activator of a costimulatory molecule, an inhibitor of an inhibitory molecule, and a vaccine.

In order to solve the above technical problem, another technical solution provided in the present invention is: the use of one or more of the antigen binding protein targeting CD40 of the present invention, the pharmaceutical composition of the present invention, the detection reagent of the present invention, the combined kits of the present invention, the genetically modified cell of the present invention and the antibody-drug conjugate of the present invention in the preparation of a drug for diagnosing, preventing and/or treating diseases such as tumors. Preferably, the tumors include solid tumors and hematologic malignancies. More preferably, the tumors include B-cell NHL, chronic lymphocytic leukemia (CLL), hairy cell leukemia (HCL), Hodgkin's disease, multiple myeloma, bladder cancer, kidney cancer, ovarian cancer, cervical cancer, breast cancer, lung cancer, nasopharyngeal cancer, malignant melanoma, pancreatic cancer and colon cancer.

In order to solve the above technical problem, another technical solution provided in the present invention is: a method for detecting CD40 in a sample, which comprises the step of contacting the sample with one or more of the antigen binding protein targeting CD40 of the present invention, the detection reagent of the present invention, and the antibody-drug conjugate of the present invention. The sample comprises, for example, a blood sample (e.g., a whole blood sample and a serum sample) and a reagent comprising CD40. Preferably, the method is for a non-diagnostic/non-therapeutic purpose, such as in scientific research, detecting the concentration of CD40 standards, whether other reagents are contaminated by CD40, etc.

In order to solve the above technical problem, another technical solution provided in the present invention is: a method for diagnosing, treating and/or preventing tumors, the method comprising administering to a patient in need thereof a therapeutically effective amount of one or more of the antigen binding protein targeting CD40 of the present invention, the isolated nucleic acid of the present invention, the recombinant expression vector of the present invention, the pharmaceutical composition of the present invention, the detection reagent of the present invention, the genetically modified cell of the present invention, and the antibody-drug conjugate of the present invention, or using the combined kits of the present invention to diagnose or treat the patient in need thereof. Preferably, the tumors include solid tumors and hematologic malignancies. More preferably, the tumors include B-cell NHL, chronic lymphocytic leukemia (CLL), hairy cell leukemia (HCL), Hodgkin's disease, multiple myeloma, bladder cancer, kidney cancer, ovarian cancer, cervical cancer, breast cancer, lung cancer, nasopharyngeal cancer, malignant melanoma, pancreatic cancer and colon cancer.

In order to solve the above technical problem, another technical solution provided in the present invention is: the use of one or more of the antigen binding protein targeting CD40 of the present invention, the isolated nucleic acid of the present invention, the recombinant expression vector of the present invention, the pharmaceutical composition of the present invention, the detection reagent of the present invention, the combined kits of the present invention, the genetically modified cell of the present invention and the antibody-drug conjugate of the present invention in the preparation of a drug for diagnosing, preventing and/or treating tumors. Preferably, the tumors include solid tumors and hematologic malignancies. More preferably, the tumors include B-cell NHL, chronic lymphocytic leukemia (CLL), hairy cell leukemia (HCL), Hodgkin's disease, multiple myeloma, bladder cancer, kidney cancer, ovarian cancer, cervical cancer, breast cancer, lung cancer, nasopharyngeal cancer, malignant melanoma, pancreatic cancer and colon cancer.

In the present invention, the term "with 3, 2 or 1 amino acid mutation" refers to including amino acid insertion, deletion or substitution occurring on the basis of the original amino acid sequence. An exemplary explanation is that the mutation of CDRs can comprise 3, 2 or 1 amino acid mutation, and the same or a different number of amino acid residues can optionally be selected for mutation between these CDRs, for example, it can be 1 amino acid mutation for CDR1, but no amino acid mutation for CDR2 and CDR3.

In the present invention, the term "PTM site mutation" refers to an amino acid mutation at the PTM site in the sequence of a variant comparing to the original amino acid sequence. Depending on different antibody sequences and different PTM sequence patterns, there are different mutation design methods. One method is to replace the "hot spot" amino acid (such as N or S in the NS pattern) with an amino acid having similar physicochemical properties (e.g., mutation of N to Q). If the PTM sequence pattern is derived from somatic hypermutation and is not present in the germline gene sequences, another method may be to replace the sequence pattern with the corresponding germline gene sequence.

In the present invention, the VH, VL or the full-length antibody can comprise mutations on the basis of the defined sequence. The mutation is a deletion, substitution or addition of one or more amino acid residues occurring in the defined amino acid sequence, and the mutated amino acid sequence has at least 85% sequence identity to the defined amino acid sequence, and maintains or improves the binding activity of the antigen binding protein comprising the mutated amino acid sequence; the at least 85% sequence identity is preferably at least 90% sequence identity; more preferably at least 95% sequence identity; most preferably at least 99% sequence identity.

In the present invention, "include", "comprise" and "is/are" have the same meaning in certain specific embodiments.

On the basis of meeting common knowledge in the art, the above-mentioned various preferred conditions can be combined in any form, such that various preferred examples of the present invention are obtained.

Reagents and raw materials used in the present invention are all commercially available.

The present invention has the positive improvement effects as follows.

The antigen binding protein targeting CD40 have properties of high affinity for CD40 and strong agonistic activity on signaling pathway, especially the enhanced agonistic activity after crosslinking, such that the antigen binding protein targeting CD40 of the present invention has a greater therapeutic window and provides a good basis for significantly improving patient response rate under safe and tolerable dosage conditions in clinical trials.

BRIEF DESCRIPTION OF THE DRAWINGS

FIG. 1A, FIG. 1B, FIG. 1C and FIG. 1D respectively show the levels of binding of some CD40 antibodies to the CHO-K1 cell highly expressing human CD40.

FIG. 2A, FIG. 2B, FIG. 2C, FIG. 2D and FIG. 2E respectively show the levels of binding of some CD40 antibodies to the Raji cell highly expressing human CD40.

FIG. 3A, FIG. 3B, FIG. 3C and FIG. 3D respectively show the levels of binding of some CD40 antibodies to the CHO-K1 cell highly expressing cynomolgus monkey CD40.

FIG. 4A, FIG. 4B, FIG. 4C and FIG. 4D respectively show the levels of the activation effects of CD40 antibody PR003379 to be tested and three control antibodies in the presence of or in the absence of crosslinking mediated by CHO-K1/hCD32B cells on HEK293-hCD40-NFkB fluorescent reporter gene cells.

FIG. 5A is the enhancement fold of fluorescence intensity of the activation of HEK293-hCD40-NFkB cells by the variant molecules of CD40 antibody PR003379 in the presence of crosslinking mediated by CHO-K1/hCD32B cells.

FIG. 5B is the enhancement fold of fluorescence intensity of the activation of HEK293-hCD40-NFkB cells by the variant molecules of CD40 antibody PR003379 in the absence of crosslinking mediated by CHO-K1/hCD32B cells.

FIG. 6A, FIG. 6B, FIG. 6C and FIG. 6D respectively show the activation effects of CD40 antibody PR003379 to be tested and three control antibodies in the presence of or in the absence of crosslinking mediated by CHO-K1/hCD32B cells on human DC cells (IL12p40 release level).

FIG. 7 shows the levels of inhibition on the binding of CD40 to CD40L by CD40 antibodies.

FIG. 8A shows the changes in tumor volume for the anti-tumor effect of CD40 antibodies in MC38-hPD-L1/CD40 humanized mouse model.

FIG. 8B shows the changes in mouse weight for the anti-tumor effect of CD40 antibodies in MC38-hPD-L1/CD40 humanized mouse model.

DETAILED DESCRIPTION OF EMBODIMENTS

The embodiments of the present invention are illustrated below by using the specific examples, and those skilled in the art would have readily understood other advantages and effects of the present invention from the content disclosed in this description.

In the present application, the term "binding protein" or "antigen binding protein" generally refers to a protein comprising a portion binding to an antigen and optionally a scaffold or framework portion that allows the antigen binding portion to adopt a conformation that promotes binding of the antigen binding protein to the antigen. It can typically comprise a light chain variable region (VL) of an antibody, a heavy chain variable region (VH) of an antibody, or both. VH and VL regions can be further divided into hypervariable regions called complementary determining regions (CDRs), which are scattered within more conserved regions called framework regions (FRs). Each VH and VL can be composed of three CDRs and four FRs, which can be arranged from an amino terminus to a carboxyl terminus in the following order: FR-1, CDR1, FR-2, CDR2, FR-3, CDR3 and FR-4. The variable regions of the heavy and light chains contain binding domains that interact with antigens. Three CDRs of VH are denoted as HCDR1, HCDR2, and HCDR3, respectively, and can also be denoted as VH CDR1, VH CDR2, and VH CDR3; and three CDRs of VL are denoted as LCDR1, LCDR2, and LCDR3, respectively, and can also be denoted as VL CDR1, VL CDR2, and VL CDR3. Examples of the antigen binding protein include, but are not limited to, an antibody, an antigen binding fragment (Fab, Fab', F(ab)$_2$, Fv fragment, F(ab')$_2$, scFv, dis-scFv and/or dAb), an immunoconjugate, a multispecific antibody (e.g., a bispecific antibody), an antibody fragment, an antibody derivative, an antibody analog, a fusion protein, and the like so long as they exhibit the desired antigen binding activity.

In the present application, the amino acid sequences of the CDRs are shown according to the rules of Chothia definition. However, it is well known to those skilled in the art that the CDRs of an antibody can be defined in the art by a variety of methods, such as the rules of Kabat definition based on sequence variability (see, Kabat et al., Sequences of Proteins of Immunological Interest, fifth edition, National Institutes of Health, Bethesda, Maryland (1991)), and the rules of Chothia definition based on the location of a structural loop region (see JMol Biol 273: 927-48, 1997). In the technical solutions of the present invention, amino acid residues in variable domain sequences can also be determined according to the rules of Combined definition that incorporates both Kabat definition and Chothia definition. The rules of Combined definition refer to the combination of the ranges of Kabat definition and Chothia definition, based on which a larger scope is taken, see Table 1-4 below for details. It should be understood by those skilled in the art that unless otherwise specified, the terms "CDR" and "complementarity determining region" of a given antibody or region thereof (for example, a variable region) should be understood to encompass the complementarity determining region as defined by any of the above-mentioned known schemes as described in the present invention. Although the scope of protection as claimed in the present invention is based on the sequences shown according to the rules of Chothia definition, corresponding amino acid sequences according to the rules of other CDR definitions shall also fall within the scope of protection of the present invention.

Table 1-4: Definition method for CDRs of antibodies of the present application

|  | Kabat | Chothia | Combined |
|---|---|---|---|
| LCDR1 | L24--L34 | L24--L34 | L24-L34 |
| LCDR2 | L50--L56 | L50--L56 | L50-L56 |
| LCDR3 | L89--L97 | L89--L97 | L89-L97 |
| HCDR1 | H31--H35 | H26--H32 | H26-H35 |
| HCDR2 | H50--H65 | H52--H56 | H50-H65 |
| HCDR3 | H95--H102 | H95--H102 | H95-H102 |

Laa-Lbb may refer to an amino acid sequence from position aa (Chothia numbering) to position bb (Chothia numbering), starting from the N-terminus of an antibody light chain; and Haa-Hbb may refer to an amino acid sequence from position aa (Chothia numbering) to position bb (Chothia numbering), starting from the N-terminus of an antibody heavy chain. For example, L24-L34 may refer to an amino acid sequence from position 24 to position 34 according to Chothia numbering, starting from the N-terminus of an antibody light chain; H26-H32 may refer to an amino acid sequence from position 26 to position 32 according to the Chothia coding rules, starting from the N-terminus of an antibody heavy chain. It should be known by those skilled in the art, when Chothia numbering scheme is used to number CDR, there may be cases where insertion sites are present at some locations (see http://bioinf.org.uk/abs/).

In the present application, the term "monoclonal antibody" generally refers to an antibody obtained from a population of substantially homogeneous antibodies, that is, individual antibodies in the population are the same, except for the possible small amount of natural mutations. The monoclonal antibody is usually highly specific for a single antigenic site. Furthermore, in contrast to a conventional polyclonal antibody preparation (which usually have different antibodies for different determinants), each monoclonal antibody is for a single determinant on the antigen. In addition to the specificity the monoclonal antibody, the monoclonal antibody has the advantage that it can be synthesized by hybridoma culture and is not contaminated by other immunoglobulins. The modifier "monoclonal" indicates the characteristic of the antibody obtained from a population of substantially homogeneous antibodies, and is not to be construed as requiring production of the antibody by any particular method. For example, the monoclonal antibody used according to the present invention can be prepared in hybridoma cells, or can be prepared by the recombinant DNA method.

In the present application, the term "fully human antibody" generally refers to an antibody expressed in a genetically engineered antibody gene-deficient animal, to which all human antibody-encoding genes are transferred. All parts of the antibody, including variable and constant regions of the antibody, are encoded by human-derived genes. The fully human antibody can greatly reduce the immune side effects caused by a heterologous antibody to the human body. Methods for obtaining the fully human antibody in the art can include phage display technique, transgenic mouse technique etc.

In the present application, the term "specific binding" and "targeting" generally refer to the binding of an antibody to an epitope by the antigen binding domain of the antibody, and the binding requires some complementarity between the antigen binding domain and the epitope. According to the definition, an antibody is said to "specifically bind" to an antigen when the antibody more likely to bind to an epitope by the antigen binding domain of the antibody than to bind to a random, unrelated epitope. The term "epitope" refers to a specific group of atoms (e.g., sugar side chain, phosphoryl and sulfonyl) or an amino acid on an antigen that binds to an antigen binding protein (e.g., an antibody).

In the present application, the term "Fab" generally refers to the antigen binding portion of a conventional antibody (e.g., IgG), including the heavy chain variable region VH, light chain variable region VL, heavy chain constant region domain CH1, and light chain constant region CL of the antibody. In a conventional antibody, the C-terminus of VH is linked to the N-terminus of CH1 to form the heavy chain Fd fragment, the C-terminus of VL is linked to the N-terminus of CL to form the light chain, and the C-terminus of CH1 is further linked to the hinge region and other constant region domains of the heavy chain to form the heavy chain. In some embodiments, "Fab" also refers to the variant structure of Fab. For example, in certain embodiments, the C-terminus of VH is linked to the N-terminus of CL to form one polypeptide chain, and the C-terminus of VL is linked to the N-terminus of CH1 to form another polypeptide chain, thereby forming a Fab (cross VH/VL) structure. In certain embodiments, CH1 of Fab is not linked to the hinge region, but the C-terminus of CL is linked to the hinge region of the heavy chain to form a Fab (cross Fd/LC) structure.

In the present application, the term "VH" generally refers to the heavy chain variable region VH domain of an antibody, that is, VH can be the heavy chain variable region VH of a conventional antibody of human or other animals (H2L2 structure), can also be the heavy chain variable region VHH of a heavy chain antibody of animal, such as camelid (HCAb structure), or can be the heavy chain variable region VH of a fully human heavy chain antibody produced by Harbour HCAb transgenic mice (HCAb structure).

In the present application, the term "CD40" generally refers to tumor necrosis factor receptor superfamily member 5 protein, a functional variant thereof and/or a functional fragment thereof, also known as TNFRSF5. The sequence of CD40 is known in the art. For example, the amino acid sequence of an exemplary human CD40 protein can be found in UniProt under the accession number of P25942; the sequence of an exemplary cynomolgus monkey CD40 protein can be found in Uniprot under the accession number of G7PG38; and the sequence of an exemplary mouse CD40 protein can be found in Uniprot under the accession number of P27512. CD40L is the natural trimeric ligand molecule of CD40.

**Examples**

The present invention is further described below by way of examples; however, the present invention is not limited to the scope of the described examples. The examples do not include a detailed description of conventional methods, such as methods for constructing vectors and plasmids, methods for inserting protein-encoding genes into such vectors and plasmids, or methods for introducing plasmids into host cells. Such methods are well known to an ordinary person skilled in the art and are described in numerous publications. For the experimental methods in which no specific conditions are specified in the following examples, selections are made according to conventional methods and conditions or according to the product instructions.

**Example 1 Preparation of CD40 antibodies**

Advances in transgenic technology allows the breeding of genetically engineered mice that carry the human immunoglobulin repertoire and in which their endogenous murine immunoglobulin repertoires are deleted. The antibody produced by this transgenic mouse has a fully human sequence, so no further humanization is required, greatly improving the efficiency of therapeutic antibody development. Harbour H2L2 mouse (Harbour antibodies BV) is a transgenic mouse carrying the human immunoglobulin repertoire, and the antibodies produced thereby have complete human antibody variable domains and rat constant domains.

Harbour H2L2 mice were immunized in multiple rounds with a soluble recombinant human CD40 extracellular domain fusion protein (Acrobiosystems, #CD0-H5253). The antigen protein was mixed with an immunoadjuvant to form an immunogen reagent, and the immunogen reagent was then injected subcutaneously in the inguinal region or injected intraperitoneally. In each round of immunization, each mouse received a total injection dose of 100 μL. In the first round of immunization, each mouse was immunized with an immunogen reagent prepared by mixing 50 μg of the antigen protein with a complete Freund's adjuvant (Sigma, #F5881) at a volume ratio of 1 : 1. In each subsequent round of booster immunization, each mouse was immunized with an immunogen reagent prepared by mixing 25 μg of the antigen protein with Sigma Adjuvant System adjuvant (Sigma, #S6322). The interval between two rounds of booster immunization was at least two weeks, typically with no more than 5 rounds of booster immunization. The immunization times were days 0, 14, 28, 42, 56, and 70; and on days 49 and 77, the antibody titers in the sera of mice were measured. Three days before the cell fusion, the final booster immunization was performed at a dose of 25 µg of the antigen protein per mouse.

When the detected titer of the CD40-specific antibody in the mouse serum reached a certain level, the mouse spleen cells were taken out and fused with the myeloma cell line to obtain hybridoma cells. After multiple rounds of screening and cloning of hybridoma cells, the hybridoma expressing the CD40 monoclonal antibody molecule was isolated. The isolated hybridoma expressed the antibody molecule having heavy and light chains of complete human variable domains and rat constant domains. The above-mentioned monoclonal antibody was further identified, and several hybridoma clones were selected for sequencing based on parameters such as the binding ability to human CD40, the binding ability to cynomolgus monkey CD40, and the ability to activate CD40 downstream signaling pathway. During the screening process, positive control antibodies listed in Table 1-5 were used (see Table 4 for the corresponding sequence numbers). The nucleotide sequence encoding the variable domain of the antibody molecule and the corresponding amino acid sequence was obtained by conventional hybridoma sequencing means. In this example, the sequences of the variable domains of the CD40 monoclonal antibody molecule obtained from the immunized Harbour H2L2 mice were human antibody sequences. The CDR sequences of the antibody variable domains could be analyzed according to Kabat or Chothia or other CDR definition rules (Table 1-5).

Table 1-5: CD40 positive control antibody

| Antibody number | Antibody name |
| --- | --- |
| PR001028 | Selicrelumab |
| PR001303 | APX005 |
| PR001304 | APX005M |
| PR001306 | CDX-1140 |

After obtaining the light and heavy chain variable domain sequences of the encoding antibody molecule, the light and heavy chain variable domain sequences can be expressed in fusion with the light and heavy chain constant domain sequences of corresponding human antibody using conventional recombinant DNA techniques to obtain recombinant antibody molecules. In this example, an antibody heavy chain variable domain (VH) sequence was obtained by gene synthesis and cloned into a mammalian cell expression plasmid vector encoding human IgG2 antibody heavy chain constant domain sequences to encode a full-length heavy chain that produces an IgG2 antibody. A light chain variable domain (VL) sequence of an antibody was obtained by gene synthesis and cloned into a mammalian cell expression plasmid vector encoding a κ light chain constant domain sequence of human antibody Ig to encode and produce a full-length light chain of an antibody. In this example, a fully human anti-CD40 recombinant IgG2 antibody was obtained.

PR006239 was obtained by gene synthesis, the nucleic acid encoding the heavy chain variable region (VH) of PR003379 was linked to the nucleic acid encoding the heavy chain constant domain sequence of an IgG1 subtype antibody containing L234A, L235A and G237A mutations and cloned into a mammalian cell expression plasmid to encode and produce a full-length heavy chain of IgG1 (L234A, L235A and G237A). The nucleic acid encoding a light chain variable domain (VL) sequence of an antibody was obtained by gene synthesis and cloned into a mammalian cell expression plasmid vector encoding a κ light chain constant domain sequence of human antibody Ig to encode and produce a full-length light chain of an antibody. The two plasmids were co-transfected into mammalian cells, and after expression, production and purification, a fully human anti-CD40 recombinant IgG1 (L234A, L235A and G237A) antibody PR006239 was obtained. See example 2 for specific antibody expression and purification methods.

**Example 2 Transient transfection expression and purification of antibodies**

In this example, the general method for preparing antibodies using techniques such as mammalian host cells (e.g., human embryonic kidney cells HEK293 or Chinese hamster ovary cells CHO and derivatives thereof), transient transfection expression and affinity capture isolation was described. The present method is suitable for an antibody of interest containing an Fc region; the antibody of interest may be composed of one or more protein polypeptide chains; and the antibody of interest may be derived from one or more expression plasmids.

The amino acid sequences of antibody polypeptide chains are converted into nucleotide sequences by codon optimization; and the nucleotide sequences for encoding are respectively synthesized and cloned onto expression vectors compatible with a host cell. The plasmids encoding the antibody polypeptide chains are simultaneously transfected into a mammalian host cell according to a particular ratio, and the recombinant antibodies having correct folding and polypeptide chain assembly can be obtained using conventional recombinant protein expression and purification techniques. Specifically, FreeStyle™ 293-F cells (Thermo, # R79007) were subjected to scale-up culture in a FreeStyle™ F17 Expression Medium (Thermo, # A1383504). Before transient transfection, the cells were adjusted to a cell concentration of 6-8 $\times$ 10$^5$ cells/ml and cultured in a shaker at 37°C and 8% $CO_2$ for 24 hours at a cell concentration of 1.2$\times$ 10$^6$ cells/ml. 30 ml of the cultured cells was prepared. The plasmids encoding the antibody polypeptide chains (pTT5, NRC) were mixed according to a certain ratio, a total of 30 $\mu$g of plasmids (the ratio of the plasmids to the cells being 1 $\mu$g : 1 ml) were dissolved in 1.5 ml of Opti-MEM reduced serum medium (Thermo, #31985088), and the resulting mixture was filtered through a 0.22 $\mu$ m filter membrane for sterilization. Another 1.5 ml of Opti-MEM was taken and dissolved in 120 $\mu$l of 1 mg/ml PEI (Polysciences, # 23966-2), and the resulting mixture was left to stand for 5 minutes. The PEI was slowly added to the plasmids, the resulting mixture was incubated at room temperature for 10 minutes, the plasmid and PEI mixed solution was slowly dripped into the culture flask while shaking the culture flask, and cultured in a shaker at 37°C and 8% $CO_2$ for 5 days. The cell viability was observed after 5 days. The culture was collected, and centrifuged at a rotary speed of 3300 g for 10 minutes, and then the supernatant was taken; and the supernatant was then centrifuged at a high speed to remove impurities. A gravity column (Bio-Rad, #7311550) containing MabSelect™ (GE Healthcare, #71-5020-91) was equilibrated with a PBS buffer with pH 7.4, that is, the column was rinsed with 2-5 times the column volume of the buffer. The supernatant sample was loaded onto the column; and the column was rinsed with 5-10 times the column volume of PBS buffer, the protein of interest was eluted with 0.1 M glycine with pH 3.5, then adjusted with Tris-HCl with pH 8.0 until neutrality, and finally transferred to a PBS buffer or a buffer containing other components through concentration and liquid exchange by an ultrafiltration tube (Millipore, # UFC901024) to obtain a purified recombinant antibody solution. Finally, the concentration was determined using NanoDrop (Thermo, NanoDrop™ One), and the purified recombinant antibody solution was subpackaged and stored for later use.

**Example 3 Sequence analysis and optimization of antibodies**

The heavy chain variable domain sequence of the antibody is derived from events such as gene rearrangement of germline gene V, D and J gene fragments of the heavy chain gene group on chromosomes and somatic hypermutation; and the light chain variable domain sequence is derived from events such as gene rearrangement of germline gene V and J gene fragments of the light chain gene group and somatic hypermutation. Gene rearrangement and somatic hypermutation are main factors that increase antibody diversity. Antibodies derived from the same germline V gene fragment may also give rise to different sequences, but the overall similarity is high. The possible germline gene fragments in the event of gene rearrangement can be deduced from the variable domain sequence of the antibody using some algorithms, such as IMGT/DomainGapAlign (http://imgt.org/3Dstructure-DB/cgi/DomainGapAlign.cgi) or NCBI/IgBLAST (https://www.ncbi.nlm.nih.gov/igblast/). The sequence of the CD40 antibody obtained in Example 1 was analyzed, with the germline V gene fragments of the heavy chain variable domain (VH) and light chain variable domain (VL) listed in Table 2.

After the translation synthesis of amino acid chains of proteins or polypeptides in cells, chemical modifications are sometimes introduced, called post-translational modifications (PTMs). If a PTM is present in a variable domain, particularly an antigen binding region (e.g., CDR) of an antibody, the presence of such a PTM can have a greater impact on the binding to an antigen, and can also cause a change in the physicochemical properties of the antibody. For example, glycosylation, deamidation, isomerization, oxidation, etc. may increase the instability or heterogeneity of antibody molecules, thereby increasing the difficulty and risk of antibody development. Avoiding some potential PTMs is thus very important for the development of therapeutic antibodies. With the accumulation of experience, it has been found that some PTMs are highly correlated with the "pattern" of the composition of amino acid sequences, particularly of the composition of adjacent amino acids, which makes it possible to predict potential PTMs from the primary amino acid sequences of proteins. Table 2 listed the predicted PTMs (NS may be a deamidation site) for the variable domains VH and VL of the antibody of Example 1.

The amino acid sequence patterns of PTMs can be disrupted by amino acid mutations, thereby reducing or removing the formation of particular PTMs. Depending on different antibody sequences and different PTM sequence patterns, there are different mutation design methods. One method is to replace the "hot spot" amino acid (such as N or S in the NS pattern) with an amino acid having similar physicochemical properties (e.g., mutation of N to Q). If the PTM sequence pattern is derived from somatic hypermutation and is not present in the germline gene sequences, another method may be to replace the sequence pattern with the corresponding germline gene sequence. In actual operation, multiple mutation design methods may be used for the same PTM sequence pattern.

Table 3 listed the new antibody molecules obtained by amino acid mutation of antibody PR003379.

Table 4 listed the sequences of the CD40 antibodies of the present invention and the amino acid sequences of the CDRs defined according to the rules of Chothia definition. These CD40 antibodies include a positive control antibody, the CD40 antibody PR003379 of the present invention, and mutant molecules thereof.

Table 2: Germline gene analysis and PTM site analysis of CD40 antibodies

| Clone No. | VH germline V gene | VL germline V gene | VH PTM | VL PTM | Recombinant antibody | Recombinant antibody subtype |
|---|---|---|---|---|---|---|
| 18C4A12-1B1 | IGHV1-2 | IGKV1-9 | | NS (LCDR3) | **PR003379** | Human IgG2 |

Table 3: Mutation site design of CD40 antibody PR003379

| Variant number | Mutation in variable region | Recombinant antibody subtype | Mutation in Fc |
|---|---|---|---|
| PR006239 | No | Human IgG1 | L234A, L235A, G237A |
| PR006492 | L:N92E | Human IgG1 | L234A, L235A, G237A |
| PR006493 | L:N92L | Human IgG1 | L234A, L235A, G237A |
| PR006494 | L:N92T | Human IgG1 | L234A, L235A, G237A |
| PR006495 | L:N92A | Human IgG1 | L234A, L235A, G237A |
| PR006496 | L:N92F | Human IgG1 | L234A, L235A, G237A |
| PR006497 | L:N92Y | Human IgG1 | L234A, L235A, G237A |
| PR006498 | L:N92I | Human IgG1 | L234A, L235A, G237A |
| PR006499 | L:N92W | Human IgG1 | L234A, L235A, G237A |
| PR006500 | L:N92M | Human IgG1 | L234A, L235A, G237A |
| PR006501 | L:N92Q | Human IgG1 | L234A, L235A, G237A |
| PR006502 | L:N92D | Human IgG1 | L234A, L235A, G237A |
| PR006503 | L:N92G | Human IgG1 | L234A, L235A, G237A |
| PR006504 | L:N92V | Human IgG1 | L234A, L235A, G237A |
| PR006505 | L:S93I | Human IgG1 | L234A, L235A, G237A |
| PR006506 | L:S93F | Human IgG1 | L234A, L235A, G237A |
| PR006507 | L:S93E | Human IgG1 | L234A, L235A, G237A |
| PR006509 | L:S93L | Human IgG1 | L234A, L235A, G237A |
| PR006510 | L:S93R | Human IgG1 | L234A, L235A, G237A |
| PR006511 | L:S93N | Human IgG1 | L234A, L235A, G237A |
| PR006512 | L:S93T | Human IgG1 | L234A, L235A, G237A |
| PR006513 | L:S93Q | Human IgG1 | L234A, L235A, G237A |
| PR006514 | L:S93V | Human IgG1 | L234A, L235A, G237A |
| PR006515 | L:S93M | Human IgG1 | L234A, L235A, G237A |
| PR006516 | L:S93H | Human IgG1 | L234A, L235A, G237A |

Table 4: Sequence numbering table of the sequences and CDR sequences (Chothia) for the CD40 antibodies of the present invention

| Antibody number | Light chain | Heavy chain | VL | VH | LCDR 1 | LCDR 2 | LCDR 3 | HCDR 1 | HCDR 2 | HCDR 3 |
|---|---|---|---|---|---|---|---|---|---|---|
| PR001028 (Selicrelumab) | 116 | 110 | 82 | 78 | 30 | 38 | 46 | 4 | 12 | 20 |
| PR001303 (APX005) | 117 | 111 | 83 | 79 | 31 | 39 | 47 | 5 | 13 | 21 |
| PR001304 (APX005M) | 117 | 112 | 83 | 79 | 31 | 39 | 47 | 5 | 13 | 21 |

(continued)

| Antibody number | Light chain | Heavy chain | VL | VH | LCDR 1 | LCDR 2 | LCDR 3 | HCDR 1 | HCDR 2 | HCDR 3 |
|---|---|---|---|---|---|---|---|---|---|---|
| PR001306 (CDX-1140) | 118 | 113 | 84 | 80 | 32 | 40 | 48 | 6 | 14 | 22 |
| PR003379 | 119 | 114 | 85 | 81 | 33 | 41 | 49 | 7 | 15 | 23 |
| PR006239 | 119 | 115 | 85 | 81 | 33 | 41 | 49 | 7 | 15 | 23 |
| PR006492 | 120 | 115 | 86 | 81 | 33 | 41 | 50 | 7 | 15 | 23 |
| PR006493 | 121 | 115 | 87 | 81 | 33 | 41 | 51 | 7 | 15 | 23 |
| PR006494 | 122 | 115 | 88 | 81 | 33 | 41 | 52 | 7 | 15 | 23 |
| PR006495 | 123 | 115 | 89 | 81 | 33 | 41 | 53 | 7 | 15 | 23 |
| PR006496 | 124 | 115 | 90 | 81 | 33 | 41 | 54 | 7 | 15 | 23 |
| PR006497 | 125 | 115 | 91 | 81 | 33 | 41 | 55 | 7 | 15 | 23 |
| PR006498 | 126 | 115 | 92 | 81 | 33 | 41 | 56 | 7 | 15 | 23 |
| PR006499 | 127 | 115 | 93 | 81 | 33 | 41 | 57 | 7 | 15 | 23 |
| PR006500 | 128 | 115 | 94 | 81 | 33 | 41 | 58 | 7 | 15 | 23 |
| PR006501 | 129 | 115 | 95 | 81 | 33 | 41 | 59 | 7 | 15 | 23 |
| PR006502 | 130 | 115 | 96 | 81 | 33 | 41 | 60 | 7 | 15 | 23 |
| PR006503 | 131 | 115 | 97 | 81 | 33 | 41 | 61 | 7 | 15 | 23 |
| PR006504 | 132 | 115 | 98 | 81 | 33 | 41 | 62 | 7 | 15 | 23 |
| PR006505 | 133 | 115 | 99 | 81 | 33 | 41 | 63 | 7 | 15 | 23 |
| PR006506 | 134 | 115 | 100 | 81 | 33 | 41 | 64 | 7 | 15 | 23 |
| PR006507 | 135 | 115 | 101 | 81 | 33 | 41 | 65 | 7 | 15 | 23 |
| PR006509 | 136 | 115 | 102 | 81 | 33 | 41 | 66 | 7 | 15 | 23 |
| PR006510 | 137 | 115 | 103 | 81 | 33 | 41 | 67 | 7 | 15 | 23 |
| PR006511 | 138 | 115 | 104 | 81 | 33 | 41 | 68 | 7 | 15 | 23 |
| PR006512 | 139 | 115 | 105 | 81 | 33 | 41 | 69 | 7 | 15 | 23 |
| PR006513 | 140 | 115 | 106 | 81 | 33 | 41 | 70 | 7 | 15 | 23 |
| PR006514 | 141 | 115 | 107 | 81 | 33 | 41 | 71 | 7 | 15 | 23 |
| PR006515 | 142 | 115 | 108 | 81 | 33 | 41 | 72 | 7 | 15 | 23 |
| PR006516 | 143 | 115 | 109 | 81 | 33 | 41 | 73 | 7 | 15 | 23 |

**Example 4 Binding to cells expressing CD40**

In this example, the binding activity of the antibody targeting CD40 to cells expressing CD40 was studied.

Flow cytometry (FACS) was used to detect the binding ability of the CD40 antibody to cells, such as, CHO-K1/hCD40 highly expressing human CD40 (constructed by Beijing Kyinno Biotechnology), CHO-K1/cyCD40 highly expressing cynomolgus monkey CD40 (constructed by Beijing Kyinno Biotechnology) and lymphoma cell highly expressing human CD40, Raji (ATCC, # CCL-86). Specifically, the cells were digested and resuspended with complete medium (F-12K for CHO-K1 cells; and RPMI-1640 for Raji cells); and the cells were adjusted to a cell density of $1 \times 10^6$ cells/mL. The cells were seeded in a 96-well V-bottom plate (Corning, #3894) at 100 $\mu$L/well, and the antigen binding protein to be tested at 2$\times$ final concentration was then added at 100 $\mu$L/well and mixed evenly, with the highest final concentration of the antigen binding protein of 300 nM, a total of 8 concentrations, and 5-fold concentration gradient dilution. hIgG (Crownbio, #C0002) was used as a negative control, and the positive control molecule was the molecule in Table 1-5. The cells were incubated at 4°C for 1 hours in the dark. After the incubation, the cells were rinsed twice by adding pre-chilled PBS at 100 $\mu$L/well, and centrifuged at 500 g at 4°C for 5 minutes, and the supernatant was discarded. Then, a fluorescent secondary antibody (goat anti-human IgG (H+L) secondary antibody, Alexa Fluor® 488 conjugate, Invitrogen, #A11013, 1 : 1000 dilution) was added at 100 μL/well, and the cells were incubated at 4°C for 30 minutes in the dark. The cells were washed twice with pre-chilled PBS at 200 μL/well, and centrifuged at 500 g at 4°C for 5 minutes, and the supernatant was discarded. Finally, the cells were resuspended in pre-chilled PBS at 200 μL/well. The fluorescence signal values were read using a BD FACS CANTOII flow cytometer or ACEA NovoCyte flow cytometer, and the data were processed and analyzed using FlowJo v10 software (FlowJo, LLC).

Data processing and graphical analysis were performed using GraphPad Prism 8 software. The binding curve of the antibody to the target cell and parameters, such as EC50 value were obtained by four-parameter nonlinear fitting.

**Example 4.1 Binding to human CD40 cell CHO-K1/hCD40**

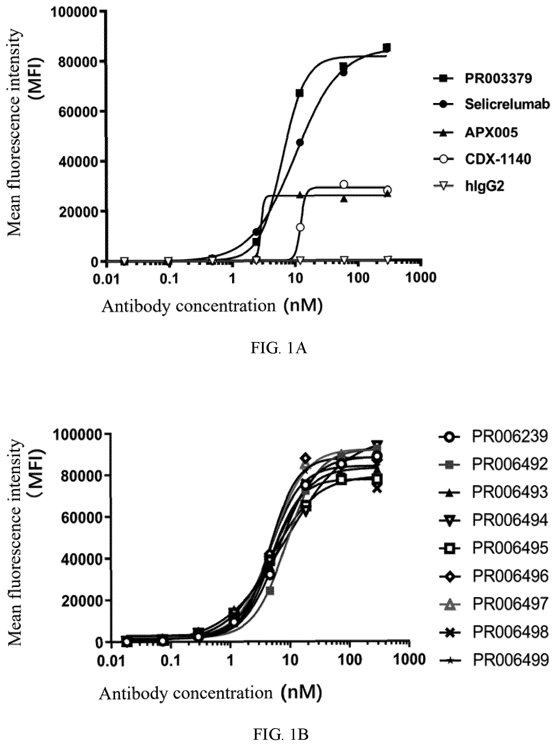

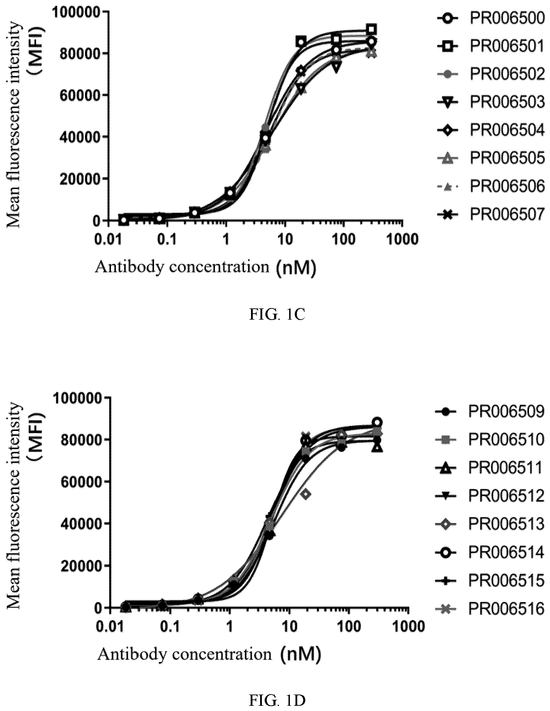

FIG. 1A, FIG. 1B, FIG. 1C and FIG. 1D and Table 5 showed the activity of CD40 antibody PR003379 and the variant molecules thereof in binding to CHO-K1/hCD40. The results showed that PR003379 and the variant molecules thereof have good binding activity to human CD40; the binding activity of PR003379 is stronger than that of the control molecule, e.g., the EC50 thereof is better than that of Selicrelumab and CDX-1140, and the maximum MFI thereof is higher than that of APX005 and CDX-1140.

Table 5: Binding of PR003379 and the variant molecules thereof to CHO-K1/hCD40 (corresponding to FIG. 1A-FIG. 1D)

| | Antibody | EC50 (nM) | Maximum MFI | | Antibody | EC50 (nM) | Maximum MFI |
|---|---|---|---|---|---|---|---|
| (A) | PR003379 | 6.345 | 81590 | (B) | PR006239 | 6.675 | 88598 |
| | Selicrelumab | 10.28 | 84982 | | PR006492 | 8.87 | 92266 |
| | APX005 | 2.831 | 25987 | | PR006493 | 5.264 | 83381 |
| | CDX-1140 | 12.2 | 29214 | | PR006494 | 8.727 | 98839 |
| | | | | | PR006495 | 4.902 | 79429 |
| | | | | | PR006496 | 4.8 | 88167 |
| | | | | | PR006497 | 5.092 | 92305 |
| | | | | | PR006498 | 4.971 | 77721 |
| | | | | | PR006499 | 4.651 | 84247 |
| | Antibody | EC50 (nM) | Maximum MFI | | Antibody | EC50 (nM) | Maximum MFI |
| (C) | PR006500 | 5.011 | 85885 | (D) | PR006509 | 5.513 | 79637 |
| | PR006501 | 5.285 | 90960 | | PR006510 | 4.981 | 82967 |
| | PR006502 | 4.553 | 88464 | | PR006511 | 4.99 | 79406 |
| | PR006503 | 6.067 | 84519 | | PR006512 | 5.182 | 81563 |
| | PR006504 | 4.873 | 85950 | | PR006513 | 7.509 | 89193 |
| | PR006505 | 6.077 | 82709 | | PR006514 | 5.079 | 86852 |
| | PR006506 | 5.853 | 82769 | | PR006515 | 4.588 | 86329 |
| | PR006507 | 5.576 | 81836 | | PR006516 | 5.08 | 86126 |

**Example 4.2 Binding to human CD40 cell Raji**

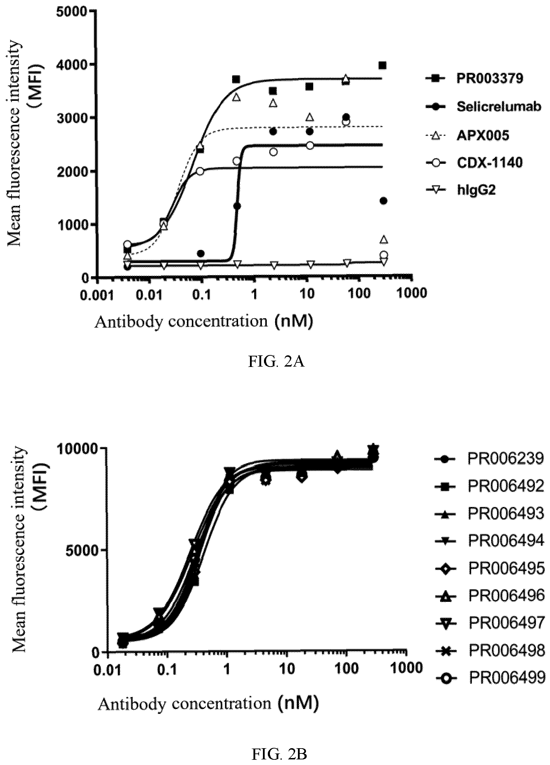

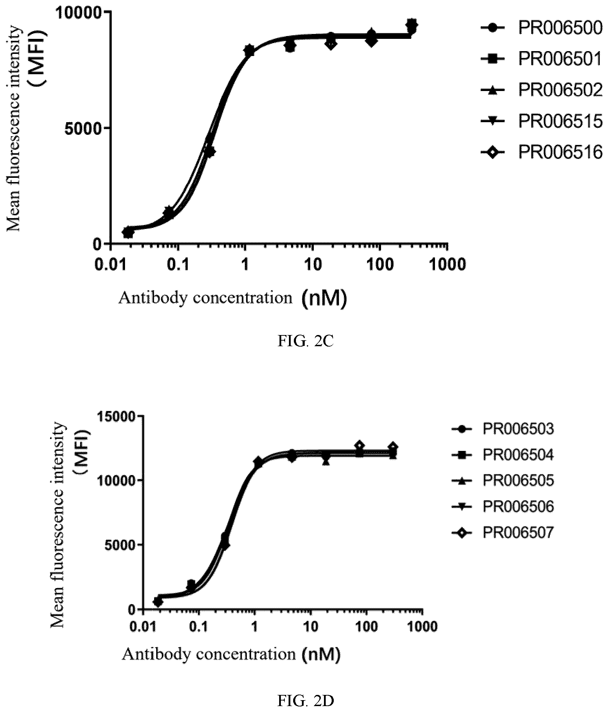

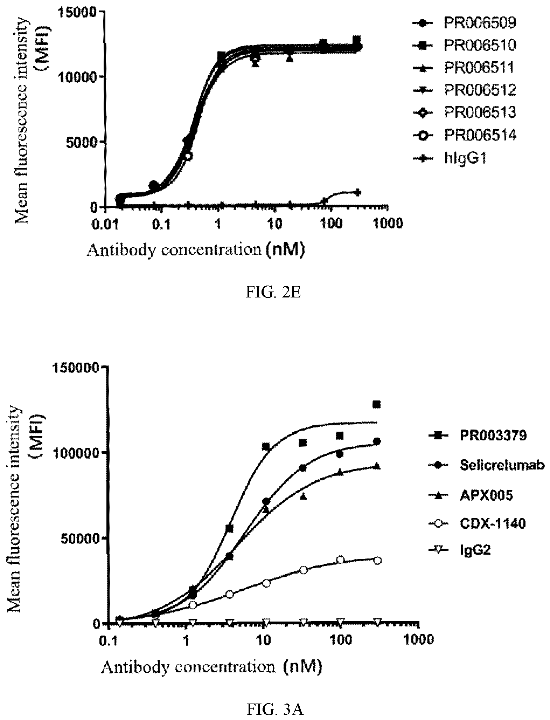

FIG. 2A, FIG. 2B, FIG. 2C, FIG. 2D and FIG. 2E and Table 6 showed the activity of CD40 antibody PR003379 and the variant molecules thereof in binding to Raji cells. The results showed that PR003379 and the variant molecules thereof could strongly bind to Raji cells; The binding activity of PR003379 was stronger than that of control molecule.

Table 6: Binging of PR003379 and the variant molecules thereof to Raji cell (corresponding to FIG. 2A-FIG. 2E)

| | Antibody | EC50 (nM) | Maximum MFI | | Antibody | EC50 (nM) | Maximum MFI |
|---|---|---|---|---|---|---|---|
| (A) | PR003379 | 0.0699 | 3696 | | | | |
| | Selicrelumab | 0.4837 | 2449 | | | | |
| | APX005 | 0.0347 | 2791 | | | | |
| | CDX-1140 | 0.028 | 614 | | | | |
| | **Antibody** | **EC50 (nM)** | **Maximum MFI** | | **Antibody** | **EC50 (nM)** | **Maximum MFI** |
| (B) | PR006239 | 0.3319 | 9048 | (C) | PR006500 | 0.3515 | 8975 |
| | PR006492 | 0.4184 | 9073 | | PR006501 | 0.3521 | 9005 |
| | PR006493 | 0.3769 | 9347 | | PR006502 | 0.2866 | 9035 |
| | PR006494 | 0.3283 | 9175 | | PR006515 | 0.3219 | 8895 |
| | PR006495 | 0.3556 | 8872 | | PR006516 | 0.3511 | 8897 |
| | PR006496 | 0.2771 | 9297 | | | | |
| | PR006497 | 0.2524 | 9203 | | | | |
| | PR006498 | 0.3443 | 8985 | | | | |
| | PR006499 | 0.3014 | 8980 | | | | |
| | **Antibody** | **EC50 (nM)** | **Maximum MFI** | | **Antibody** | **EC50 (nM)** | **Maximum MFI** |
| (D) | PR006503 | 0.3328 | 12171 | (E) | PR006509 | 0.371 | 12204 |
| | PR006504 | 0.3503 | 12152 | | PR006510 | 0.3762 | 12373 |
| | PR006505 | 0.336 | 11935 | | PR006511 | 0.3859 | 11785 |
| | PR006506 | 0.3879 | 12128 | | PR006512 | 0.4241 | 12067 |
| | PR006507 | 0.3836 | 12332 | | PR006513 | 0.3734 | 11986 |
| | | | | | PR006514 | 0.4546 | 12037 |

**Example 4.3 Binding to cynomolgus monkey CD40 cell CHO-K1/cyCD40**

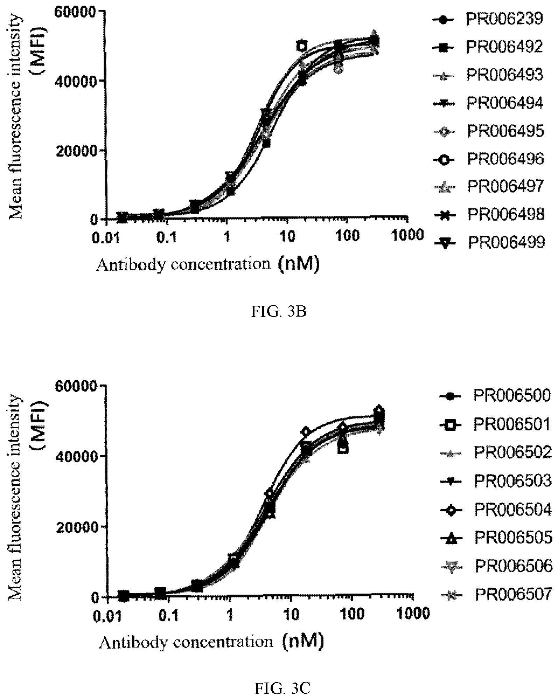

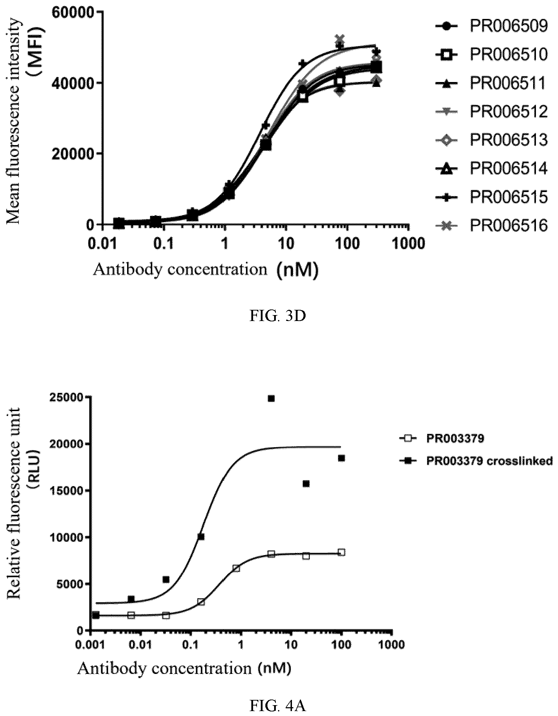

FIG. 3A, FIG. 3B, FIG. 3C and FIG. 3D and Table 7 showed the activity of CD40 antibody PR003379 and the variant molecules thereof in binding to cynomolgus monkey CD40 cell CHO-K1/cyCD40. The results showed that PR003379 and the variant molecules thereof had good binding activity to cynomolgus monkey CD40; The binding activity of PR003379 was stronger than that of control molecule.

Table 7: Binding of PR003379 and the variant molecules thereof to CHO-K1/cyCD40 (corresponding to FIG. 3A-FIG. 3D)

| | Antibody | EC50 (nM) | Maximum MFI | | Antibody | EC50 (nM) | Maximum MFI |
|---|---|---|---|---|---|---|---|
| (A) | PR003379 | 3.878 | 116864 | (B) | PR006239 | 4.408 | 51824 |
| | Selicrelumab | 5.97 | 105580 | | PR006492 | 6.256 | 52166 |
| | APX005 | 4.721 | 93571 | | PR006493 | 4.061 | 48943 |
| | CDX-1140 | 5.536 | 39735 | | PR006494 | 4.042 | 49590 |
| | | | | | PR006495 | 4.406 | 48028 |
| | | | | | PR006496 | 3.503 | 49738 |
| | | | | | PR006497 | 3.544 | 51708 |
| | | | | | PR006498 | 4.045 | 47335 |
| | | | | | PR006499 | 3.199 | 49776 |

(continued)

| | Antibody | EC50 (nM) | Maximum MFI | | Antibody | EC50 (nM) | Maximum MFI |
|---|---|---|---|---|---|---|---|
| (C) | PR006500 | 4.403 | 48505 | (D) | PR006509 | 4.693 | 45342 |
| | PR006501 | 4.189 | 48203 | | PR006510 | 4.554 | 44305 |
| | PR006502 | 3.961 | 47725 | | PR006511 | 3.684 | 40321 |
| | PR006503 | 3.988 | 49554 | | PR006512 | 4.546 | 45792 |
| | PR006504 | 3.71 | 51016 | | PR006513 | 3.564 | 40200 |
| | PR006505 | 4.619 | 48059 | | PR006514 | 4.233 | 45078 |
| | PR006506 | 4.504 | 47308 | | PR006515 | 3.745 | 50571 |
| | PR006507 | 5.009 | 49256 | | PR006516 | 5.293 | 51388 |

**Example 5 Affinity for CD40 protein**

To detect the affinity of CD40 antibodies for antigens such as human CD40 protein, the binding kinetics between antigen and antibody was analyzed using an Octet Red 96e (Fortebio) molecular interaction analyzer by biolayer interferometry (BLI) technique.

10×kinetics buffer (ForteBio, # 18-1105) was first diluted to 1×kinetics buffer for affinity testing and dilution of antigens and antibodies. For determining the affinity of antibodies for antigens, the rotary speed of the sensor was set at 1000 rpm. AHC sensors (Fortebio, # 18-5060) placed in one column were first equilibrated in the above test buffer diluted to 1× for 10 minutes, and then used to capture the CD40 antibodies at a capture height of 0.85 nm; and after being equilibrated in the buffer for 120 s, the AHC sensors bound to human CD40 protein with 2-fold gradient dilution (the concentration range of CD40 for binding to PR003379 and CDX-1140 being 20-5 nM and 0 nM; the concentration range of CD40 for binding to APX005M and Selicrelumab being 120 to 30 nM and 0 nM) for 180 s and dissociated for 500 s. The AHC sensors were finally regenerated by immersing in a 10 mM glycine-hydrochloric acid solution with pH 1.5 to elute the protein bound to the sensors.

For data analysis using Octet Data Analysis software (Fortebio, version 11.0), 0 nM well was used as the reference well, reference subtraction was performed, "1 : 1 Global fitting" method was selected for data fitting, the kinetic parameters of the binding of the antigen to the CD40 antibodies were calculated, and the kon (1/Ms) value, kdis (1/s) value and KD (M) value were obtained.

Affinity test results are shown in Table 8, PR003379 bound to human CD40 protein with a strong affinity (about 138 pM), and the affinity was stronger than that of the positive control antibody.

Table 8: Affinity of CD40 antibodies for binding to human CD40 protein

| Antibody | Antigen concentration | KD (M) | kon (1/Ms) | kdis (1/s) | Full R^2 |
|---|---|---|---|---|---|
| PR003379 | 20 - 5 nM | 1.38E-10 | 1.04E+06 | 1.44E-04 | 0.9703 |
| Selicrelumab | 120 - 30 nM | 4.50E-09 | 1.07E+05 | 4.80E-04 | 0.9746 |
| APX005M | 120 - 30 nM | 7.14E-09 | 3.69E+05 | 2.64E-03 | 0.9909 |
| CDX-1140 | 20 - 5 nM | 5.25E-10 | 1.09E+06 | 5.70E-04 | 0.9939 |

**Example 6 Activity in fluorescence reporter gene experiments**

In this example, fluorescent reporter gene experiments were used to study the activation effect of CD40 antibodies in the presence of or in the absence of Fc crosslinking on CD40. Cells highly expressing human CD32B were used to perform Fc-mediated crosslinking of the antibodies, to study whether crosslinking could further enhance the ability to activate CD40.

CHO-K1 cells expressing human CD32B (CHO-K1/hCD32B, Genscript, #M00600) and CHO-K1 cells were plated at $1 \times 10^4$/well, 100 μL/well in a 96-well plate (PerkinElmer, #6005181) and incubated at 37°C in 5% $CO_2$ environment overnight. The supernatant was removed, and HEK293-hCD40-NFkB reporter gene cells expressing human CD40 and NFkB-luciferase reporter gene (BPS bioscience, #60626) were collected and added to a 96-well plate at $5 \times 10^4$/well or 50 μL/well. 50 μL/well of the dilution solution of the antibody protein to be tested was added with an initial concentration of 100 nM, 5-fold concentration dilution and a total of 8 concentrations. Culture was performed at 37°C in 5%

$CO_2$ environment for 6 hours. ONE-Glo™ luciferase reagent (Promega, #E6110) was added, incubation was performed at room temperature for 5 minutes, and the luminescence value was detected by a microplate reader (PerkinElmer EnSpire). Data processing and graphical analysis were performed using GraphPad Prism 8 software. The curve of the antibody concentration-dependent relative fluorescence signal unit (RLU) and parameters, such as EC50 value were obtained by four-parameter nonlinear fitting. The sample in which the CD40 antibody was incubated with CHO-K1/hCD32B cells was referred to as "crosslinked"; and the sample in which the CD40 antibody was incubated with CHO-K1 cells was referred to as "uncrosslinked".

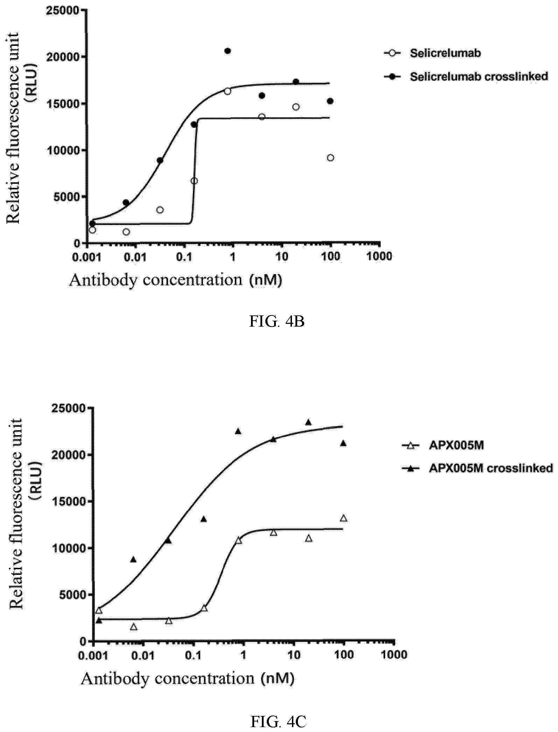

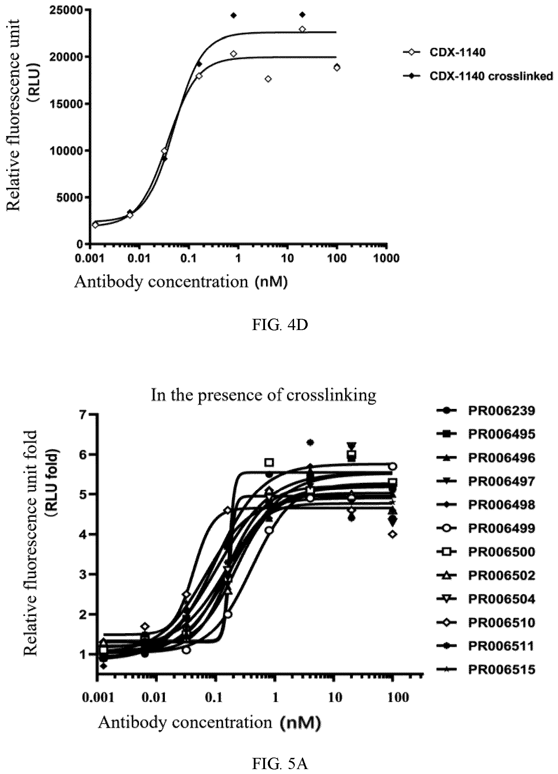

As shown in FIG. 4A, FIG. 4B, FIG. 4C and FIG. 4D and Table 9, PR003379 and control antibody APX005M had an obvious "crosslinking enhancement" effect, that is, PR003379 and APX005M in the presence of CD32B-mediated antibody crosslinking could significantly amplify the signal of the fluorescent reporter gene, indicating that the activation effect of the CD40 molecule on the downstream signal molecule was significantly enhanced.

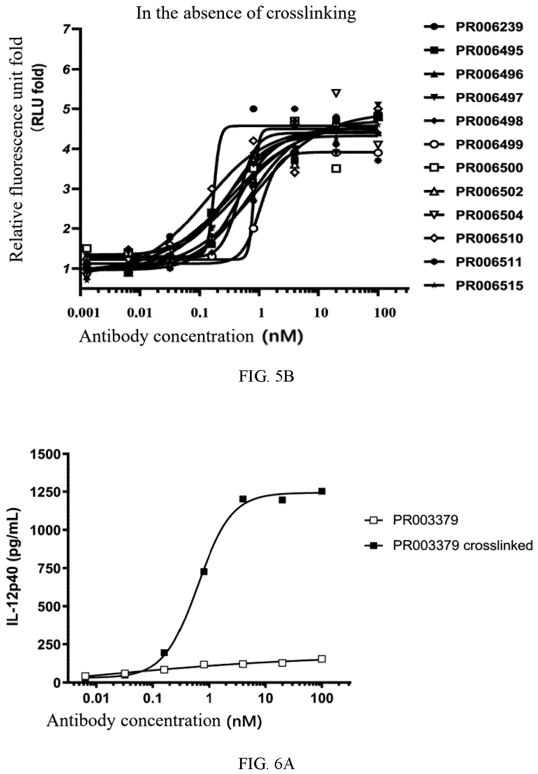

FIG. 5A and FIG. 5B and Table 10 showed the enhancement fold of fluorescence intensity of the activation of reporter gene cells by the variant molecules of PR003379 in the presence of or in the absence of crosslinking mediated by CD32B cells. The results showed that the variant molecules of PR003379 in the presence of crosslinking could significantly enhance the activation effect on CD40 molecule, which was manifested in that the EC50 value in the presence of crosslinking was significantly reduced compared with the EC50 value in the absence of crosslinking (expressed as "fold (EC50 in the absence of crosslinking/EC50 in the presence of crosslinking)"> 1).

The RLU fold/fold in Table 9 and Table 10 represented the difference in activation potential of the antibody under two conditions, or the therapeutic window, and reflected that PR003379 and the derivative antibodies thereof had better folds than that of the existing antibody and had a larger therapeutic window.

Table 9: Activity of CD40 antibody for activating the HEK293-CD40-NFkB reporter gene cell

| Antibody | EC50 (nM) | Maximum RLU | RLU fold (crosslinked/uncrosslinked) |
|---|---|---|---|
| PR003379 | 0.3693 | 8252 | 2.38 |
| PR003379 crosslinked | 0.1784 | 19664 | |
| Selicrelumab | 0.1628 | 13355 | 1.28 |
| Selicrelumab crosslinked | 0.0413 | 17036 | |
| APX005M | 0.3551 | 11898 | 1.95 |
| APX005M crosslinked | 0.0420 | 23207 | |
| CDX-1140 | 0.0371 | 19955 | 1.13 |
| CDX-1140 crosslinked | 0.0506 | 22605 | |

Table 10: Activity of PR003379 variants in the presence of or in the absence of crosslinking for activating the reporter gene cell

| PR003379 variant | Crosslinked | | Uncrosslinked | | Fold (EC50 in the absence of crosslinking/EC50 in the presence of crosslinking) |
|---|---|---|---|---|---|
| | EC50 (nM) | Maximum fold (fold) | EC50 (nM) | Maximum fold (fold) | |
| PR006239 | 0.0980 | 5.76 | 0.1734 | 4.58 | 1.77 |
| PR006495 | 0.1092 | 5.54 | 0.9047 | 4.92 | 8.28 |
| PR006496 | 0.2172 | 5.03 | 0.4296 | 4.43 | 1.98 |
| PR006497 | 0.1582 | 4.77 | 0.4786 | 4.61 | 3.03 |
| PR006498 | 0.1958 | 5.53 | 0.4615 | 4.4 | 2.36 |
| PR006499 | 0.4054 | 5.28 | 1.0440 | 3.92 | 2.58 |
| PR006500 | 0.1689 | 5.55 | 0.3012 | 4.33 | 1.78 |
| PR006502 | 0.1704 | 4.95 | 0.3212 | 4.63 | 1.88 |
| PR006504 | 0.1572 | 5.21 | 0.7079 | 4.7 | 4.50 |
| PR006510 | 0.0423 | 4.66 | 0.1253 | 4.54 | 2.96 |

(continued)

| PR003379 variant | Crosslinked | | Uncrosslinked | | |
|---|---|---|---|---|---|
| | EC50 (nM) | Maximum fold (fold) | EC50 (nM) | Maximum fold (fold) | Fold (EC50 in the absence of crosslinking/EC50 in the presence of crosslinking) |
| PR006511 | 0.1600 | 5.19 | 0.8153 | 4.5 | 5.10 |
| PR006515 | 0.0669 | 4.91 | 0.2683 | 4.54 | 4.01 |

**Example 7 Activity in DC cell activation experiments**

In this example, cells highly expressing human CD32B were used to perform Fc-mediated crosslinking of the antibodies, to study whether crosslinking could further enhance the ability of CD40 antibodies to activate DC cells.

CHO-K1 cells expressing human CD32B (CHO-K1/hCD32B) and CHO-K1 cells were plated at $1 \times 10^4$/well or 50 $\mu$L/well in a 96-well plate (Corning, #3599), respectively. 50 $\mu$L/well of the dilution solution of the antibody protein to be tested was added with an initial concentration of 100 nM, 5-fold concentration dilution and a total of 8 concentrations. Induced mature DC cells isolated from human PBMCs were added at $2 \times 10^4$/well or 100 $\mu$L/well, and incubated in an incubator at 37°C and 5% $CO_2$ for 2 days. The supernatant on the second day was collected, and the content of IL-12p40 in the supernatant was detected using the human IL-12p40 ELISA kit (R&D system, #DY240). Data processing and graphical analysis were performed using GraphPad Prism8 software. The curve of the antibody concentration-dependent IL12p40 release level and parameters, such as EC50 value were obtained by four-parameter nonlinear fitting. The sample in which the CD40 antibody was incubated with CHO-K1/hCD32B cells was referred to as "crosslinked"; and the sample in which the CD40 antibody was incubated with CHO-K1 cells was referred to as "uncrosslinked".

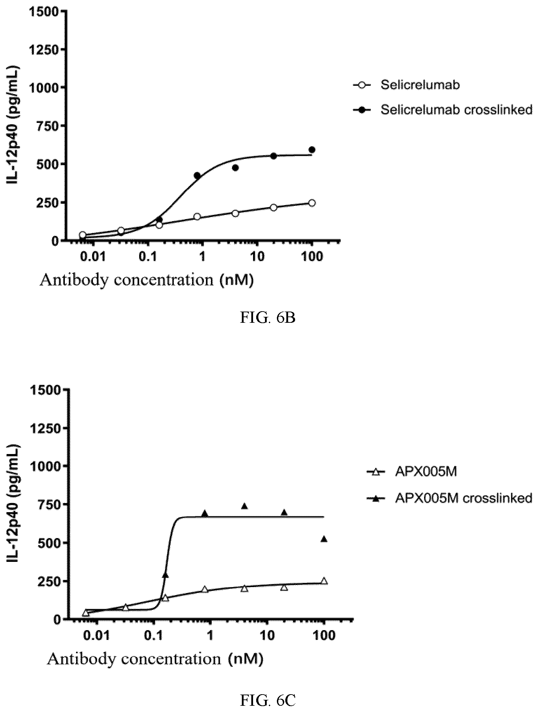

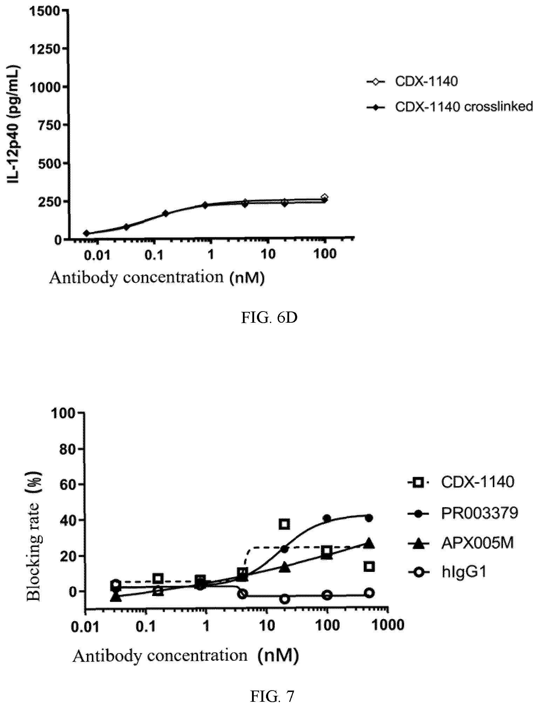

The results are shown in FIG. 6A, FIG. 6B, FIG. 6C and FIG. 6D and Table 11, PR003379 had an obvious "crosslinking enhancement" effect, that is, PR003379 in the absence of CD32B-mediated antibody crosslinking activated DC cells to release limited levels of IL-12p40, however, PR003379 in the presence of CD32B-mediated antibody crosslinking could significantly enhance activation of DC cells, and maximum IL-12p40 could reach 1243.0 pg/mL, which was much higher than that of other control antibodies.

Table 11: Activation of CD40 antibodies on DC cells to release cytokine IL-12p40

| Antibody | EC50 (nM) | Maximum IL12p40 (pg/mL) | Fold of maximum IL12p40 (crosslinked/uncrosslinked) |
|---|---|---|---|
| PR003379 | 0.0102 | 179.2 | 6.94 |
| PR003379 crosslinked | 0.6268 | 1243.0 | |
| Selicrelumab | 0.3800 | 318.7 | 1.75 |
| Selicrelumab crosslinked | 0.3917 | 558.9 | |
| APX005M | 0.0735 | 242.5 | 2.75 |
| APX005M crosslinked | 0.1695 | 666.1 | |
| CDX-1140 | 0.0889 | 252.1 | 0.92 |
| CDX-1140 crosslinked | 0.0886 | 231.6 | |

**Example 8 Blocking the binding of CD40 and CD40L**

To detect the effect of CD40 antibodies on the binding of CD40 to CD40L, CHO-K1/hCD40 cells were plated at $2 \times 10^5$/well or 100 $\mu$L/well into a 96-well plate (Corning, #3799) and centrifuged, and the supernatant was taken and the dilution solution of the antigen binding protein to be tested was added at 50 $\mu$L/well with an initial concentration of 500 nM, 5-fold concentration dilution and a total of 8 concentrations. 20 ug/mL of human CD40L-his protein (Sino biological, #10239-H08E) was added at 50 $\mu$L/well, the cells were incubated at 4 °C for 1 hour and then washed 3 times with FACS buffer (1xPBS with 2%FBS). 1 : 400 diluted anti-histidine tag secondary antibody (GenScript, #A01800) was added, and the cells were incubated at 4 °C for 1 hour, then washed 2 times with FACS buffer, and resuspended in pre-chilled PBS. The fluorescence signal values were read using a BD FACS CANTOII flow cytometer or ACEA NovoCyte flow cytometer, and the data were processed and analyzed using FlowJo v10 software (FlowJo, LLC).

Data processing and graphical analysis were performed using GraphPad Prism 8 software. The curve of the antibody concentration-dependent inhibition rate and parameters, such as IC50 value were obtained by four-parameter nonlinear fitting.

As shown in FIG. 7 and Table 12, PR003379 exhibited partial inhibition of the binding of CD40 to CD40L with a maximum inhibition rate of 41.9%.

Table 12: Inhibition of the binding of CD40 to CD40L by CD40 antibodies

| Antibody | IC50 (nM) | Maximum inhibition rate (%) |
|---|---|---|
| CDX-1140 | 4.4 | 24.0 |
| PR003379 | 17.0 | 41.9 |
| APX005M | 10.0 | 26.0 |
| hIgG1 | No | 2.3 |

**Example 9 Determination of competition of antibodies for binding to CD40 epitopes by BLI method**

To detect binding epitopes of CD40 antibodies, epitope competition experiments were performed on CD40 antibodies Selicrelumab, APX005M and PR003379 using Octet Red 96e (Fortebio) molecular interaction analyzer by BLI technique. First step, acquisition of 100% signal of the antibody: the CD40 protein (Sino Biological, #10774-H08H) was captured using HIS1K sensors (Fortebio, #18-5120) at a capture height of 0.2 nm; after being equilibrated in a buffer for 120 s, the sensors were immersed in various antibodies diluted to 140 nM for 220 s, and the final signal of the antibody binding to CD40 was recorded as the 100% signal for that antibody. Second step, epitope competition: the CD40 protein was captured using HIS1K sensors at a capture height of 0.2 nm; the sensors were immersed in a first antibody (concentration: 140 nM) for 220 s, then the HIS1K sensors were immersed in a mixture of the first antibody and a second antibody (final concentration of both antibodies: 140 nM) for 220 s, and the difference in signals before and after immersing the sensors in the mixture of the two antibodies was recorded as the signal for that antibody as the second antibody. The inhibition rate was calculated by the following equation,

$$\text{Inhibition rate (\%)} = (A - B) / A * 100$$

A: 100% signal (obtained from the first step) of a certain antibody, B: signal (obtained from the second step) of this antibody as the second antibody.

If the resulting inhibition rate is greater than 80 %, it means that the epitopes to which the two antibodies bind completely overlap; if the inhibition rate is greater than 40% and less than 80%, it means that the epitopes to which the two antibodies bind do not completely overlap or the epitopes are very close to each other; and if the inhibition rate is less than 40%, it means that the epitopes for the two antibodies are different.

The results are shown in Table 13, the binding epitopes for PR003379 and Selicrelumab are almost identical, however the binding epitopes for PR003379 and APX005M are different.

Table 13: BLI epitope competition experiment

| Inhibition rate (%) | | Second antibody | | |
|---|---|---|---|---|
| | | Selicrelumab | APX005M | PR003379 |
| First antibody | Selicrelumab | 95.11 | 10.92 | 92.8 |
| | APX005M | 0.53 | 98.59 | -0.12 |
| | PR003379 | 98.87 | 9.56 | 96.74 |

**Example 10 Anti-tumor activity and effect on mouse weight in MC38-hPD-L1/hCD40-C57BL/6J mouse model**

To detect the anti-tumor function of CD40 antibodies in vivo, MC38 mouse colon cancer cells overexpressing human PD-L1 (MC38/hPD-L1, provided by Shanghai Model Organisms Center, Inc.) were cultured in an incubator at 37°C and 5% $CO_2$, where the medium was DMEM medium containing 10% inactivated fetal bovine serum. The cells grew to fill the culture dish every 3 to 4 days, and then were splited to separate dish(s) for subculture. MC38/hPD-L1 in the logarithmic growth phase was taken, resuspended in PBS, counted, and adjusted to a cell concentration of $1.0 \times 10^7$/mL. The cell

suspension was inoculated subcutaneously in the right lateral thorax of CD40 humanized transgenic mice (hCD40-C57BL/6J, provided by Shanghai Model Organisms Center, Inc.) at 100 $\mu$L/mouse using a 1 mL syringe, with about $1.0 \times 10^6$ tumor cells inoculated per mouse. When the mean tumor volume reached 114 mm$^3$, mice with a moderate tumor volume were picked and randomly divided to each experimental group according to tumor volumes, 6 mice per group. The administration began on the day of grouping.

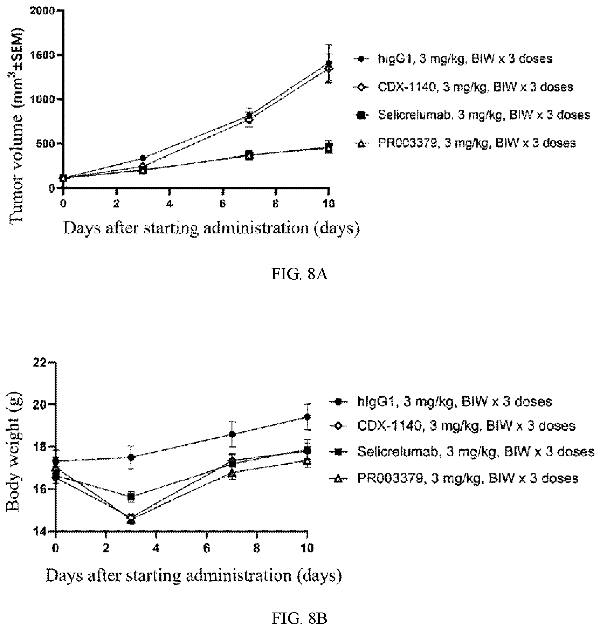

The results are shown in FIG. 8A and FIG. 8B. On day 10 after starting administration, the average tumor volume of the control group IgG1 (3 mg/kg) was 1409.41 $\pm$ 205.02 mm$^3$. The average tumor volumes of Selicrelumab (3 mg/kg) group, CDX-1104 (3 mg/kg) group and PR003379 (3 mg/kg) group were 460.96 $\pm$ 68.61 mm$^3$, 1345.22$\pm$162.94 mm$^3$ and 447.89 $\pm$ 50.74 mm$^3$, respectively, and the tumor inhibition rates were 73.22%, 4.92% and 74.24%, respectively. During the whole experiment, body weight growth rates of mice in IgG1 control group, Selicrelumab group, CDX-1104 group and PR003379 group were 12.24%, 7.31%, 7.56% and 1.86%, respectively. The mice were in normal condition.

Although the specific embodiments of the present invention have been described above, it will be understood by those skilled in the art that these are merely illustrative, and that various alterations or modifications can be made to these embodiments without departing from the principle and essence of the present invention. Therefore, the scope of protection of the present invention is defined by the appended claims.

### Claims

1. An antigen binding protein targeting CD40, which comprises a light chain variable region (VL) comprising LCDR1, LCDR2, and LCDR3 and a heavy chain variable region (VH) comprises HCDR1, HCDR2, and HCDR3; wherein

   the LCDR1 comprises an amino acid sequence set forth in SEQ ID NO: 33, the LCDR2 comprises an amino acid sequence set forth in SEQ ID NO: 41, the LCDR3 comprises an amino acid sequence set forth in SEQ ID NO: 49 or a variant 3 with 3, 2 or 1 amino acid mutation in SEQ ID NO: 49; the HCDR1 comprises an amino acid sequence set forth in SEQ ID NO: 7, the HCDR2 comprises an amino acid sequence set forth in SEQ ID NO: 15, and the HCDR3 comprises an amino acid sequence set forth in SEQ ID NO: 23;
   preferably, the variant 3 comprises an amino acid sequence having a PTM site mutation in the amino acid sequence set forth in SEQ ID NO: 49, preferably comprises an amino acid sequence having an amino acid mutation at position 4 and/or position 5 of the amino acid sequence set forth in SEQ ID NO: 49, the amino acid mutation is preferably an amino acid substitution, and more preferably a conservative amino acid substitution.

2. The antigen binding protein targeting CD40 of claim 1, wherein the variant 3 is an amino acid sequence set forth in any one of SEQ ID NOs: 50-73;
   preferably, the LCDR1 comprises an amino acid sequence set forth in SEQ ID NO: 33, the LCDR2 comprises an amino acid sequence set forth in SEQ ID NO: 41, the LCDR3 comprises an amino acid sequence set forth in any one of SEQ ID NOs: 49-73, the HCDR1 comprises an amino acid sequence set forth in SEQ ID NO: 7, the HCDR2 comprises an amino acid sequence set forth in SEQ ID NO: 15, and the HCDR3 comprises an amino acid sequence set forth in SEQ ID NO: 23.

3. The antigen binding protein targeting CD40 of claim 1 or 2, wherein the light chain variable region comprises an amino acid sequence set forth in SEQ ID NO: 85 or an amino acid sequence having at least 85%, 90%, 92%, 94%, 95%, 96%, 97%, 98% or 99% identity to SEQ ID NO: 85, and the heavy chain variable region comprises an amino acid sequence set forth in SEQ ID NO: 81 or an amino acid sequence having at least 85%, 90%, 92%, 94%, 95%, 96%, 97%, 98% or 99% identity to SEQ ID NO: 81; preferably, the light chain variable region comprises an amino acid sequence set forth in any one of SEQ ID NOs: 85-109, and the heavy chain variable region comprises an amino acid sequence set forth in SEQ ID NO: 81.

4. The antigen binding protein targeting CD40 of any one of claims 1-3, wherein the antigen binding protein targeting CD40 satisfies at least one of the following three conditions:

   (1) the antigen binding protein targeting CD40 is a full-length antibody, Fab, Fab', F(ab')2, or Fv, and preferably the Fv is scFv;
   (2) the antigen binding protein targeting CD40 is a monospecific antibody, a bispecific antibody, or a multispecific antibody; and
   (3) the antigen binding protein targeting CD40 is a monoclonal antibody or a polyclonal antibody; preferably, the antigen binding protein targeting CD40 is a full-length antibody comprising a light chain and a heavy chain, the light chain comprises a light chain constant region (CL), the light chain constant region is preferably a light chain constant region of human antibody, and the heavy chain comprises a heavy chain constant region (CH), and the heavy chain constant region is preferably a heavy chain constant region of human antibody, more preferably a heavy chain constant region of hIgG1, hIgG2, hIgG3 or hIgG4 subtype, and further preferably a heavy chain constant region of hIgG1 subtype.

5. The antigen binding protein targeting CD40 of any one of claims 1-4, wherein the antigen binding protein targeting CD40 is a full-length antibody comprising a light chain and a heavy chain, the heavy chain comprises an amino acid sequence set forth in SEQ ID NO: 114 or 115, and the light chain comprises an amino acid sequence set forth in any one of SEQ ID NOs: 119-143; preferably, the heavy chain comprises an amino acid sequence set forth in SEQ ID NO: 114, and the light chain comprises an amino acid sequence set forth in SEQ ID NO: 119, or, the heavy chain comprises an amino acid sequence set forth in SEQ ID NO: 115, and the light chain comprises an amino acid sequence set forth in any one of SEQ ID NOs: 119-143.

6. A chimeric antigen receptor, wherein the chimeric antigen receptor comprises the antigen binding protein targeting CD40 of any one of claims 1-5.

7. An isolated nucleic acid, wherein the isolated nucleic acid encodes the antigen binding protein targeting CD40 of any one of claims 1-5 or the chimeric antigen receptor of claim 6.

8. A recombinant expression vector, wherein the recombinant expression vector comprises the isolated nucleic acid of claim 7; preferably, the recombinant expression vector comprises a eukaryotic cell expression vector and/or a prokaryotic cell expression vector.

9. A transformant, wherein the transformant comprises the isolated nucleic acid of claim 7 or the recombinant expression vector of claim 8; preferably, a host cell of the transformant is a prokaryotic cell and/or a eukaryotic cell, the prokaryotic cell is preferably an *E. coli* cell such as a TG1 or a BL21, and the eukaryotic cell is preferably an HEK293 cell or a CHO cell.

10. A method for preparing the antigen binding protein targeting CD40 of any one of claims 1-5, wherein the method comprises the following steps: culturing the transformant of claim 9, and obtaining the antigen binding protein targeting CD40 from the culture.

11. An antibody-drug conjugate, wherein the antibody-drug conjugate comprises the antigen binding protein targeting CD40 of any one of claims 1-5, and a cytotoxic agent or a label; preferably, the cytotoxic agent is MMAF or MMAE, and the label is a fluorescent agent.

12. A genetically modified cell, wherein the genetically modified cell expresses the chimeric antigen receptor of claim 6; preferably, the genetically modified cell is a eukaryotic cell, preferably an isolated human cell, more preferably an immune cell such as T cell or NK cell.

13. A pharmaceutical composition, wherein the pharmaceutical composition comprises one or more of the antigen binding protein targeting CD40 of any one of claims 1-5, the isolated nucleic acid of claim 7, the recombinant expression vector of claim 8, the antibody-drug conjugate of claim 11 and the genetically modified cell of claim 12, and a pharmaceutically acceptable carrier and/or a pharmaceutically acceptable adjuvant; preferably, the pharmaceutical composition further comprises other anti-tumor antibodies as active ingredients.

14. A detection reagent, wherein the detection reagent comprises the antigen binding protein targeting CD40 of any one of claims 1-5 and/or the antibody-drug conjugate of claim 11; preferably, the detection reagent is in a liquid dosage form, a gaseous dosage form, a solid dosage form and a semi-solid dosage form; more preferably, the detection reagent further comprises a secondary antibody, CD40 or a derivative thereof, the secondary antibody is, for example, an anti-human IgG antibody conjugated to horseradish peroxidase and an anti-human IgG antibody conjugated to biotin.

15. A combined kits, wherein the combined kits comprise kit A containing one or more of the antigen binding protein targeting CD40 of any one of claims 1-5, the pharmaceutical composition of claim 13, the detection reagent of claim 14, the antibody-drug conjugate of claim 11 and the genetically modified cell of claim 12;
preferably, the combined kits further comprise kit B containing other anti-tumor antibodies or a pharmaceutical composition comprising the other anti-tumor antibodies, and/or one or more selected from the group consisting of a hormone preparation, a targeting small molecule preparation, a proteasome inhibitor, an imaging agent, a diagnostic agent, a chemotherapeutic agent, an oncolytic drug, a cytotoxic agent, a cytokine, an activator of a costimulatory molecule, an inhibitor of an inhibitory molecule, and a vaccine.

16. Use of one or more of the antigen binding protein targeting CD40 of any one of claims 1-5, the isolated nucleic acid of claim 7, the recombinant expression vector of claim 8, the pharmaceutical composition of claim 13, the detection reagent of claim 14, the combined kits of claim 15, the antibody-drug conjugate of claim 11 and the genetically modified cell of claim 12 in the preparation of a drug for diagnosing, preventing and/or treating tumors; preferably, the tumors include solid tumors and hematologic malignancies; more preferably, the tumors include B-cell NHL, chronic lymphocytic leukemia (CLL), hairy cell leukemia (HCL), Hodgkin's disease, multiple myeloma, bladder cancer, kidney cancer, ovarian cancer, cervical cancer, breast cancer, lung cancer, nasopharyngeal cancer, malignant melanoma, pancreatic cancer and colon cancer.

17. A method for detecting CD40 in a sample, wherein the method comprises the step of contacting the sample with the antigen binding protein targeting CD40 of any one of claims 1-5, the detection reagent of claim 14 and/or the antibody-drug conjugate of claim 11; preferably, the method is for non-diagnostic and/or non-therapeutic purposes.

18. A method for diagnosing, treating and/or preventing tumors, the method comprising administering to a patient in need thereof a therapeutically effective amount of one or more of the antigen binding protein targeting CD40 of any one of claims 1-5, the isolated nucleic acid of claim 7, the recombinant expression vector of claim 8, the pharmaceutical composition of claim 13, the detection reagent of claim 14, the combined kits of claim 15, the antibody-drug conjugate of claim 11 and the genetically modified cell of claim 12, or using the combined kits of claim 15 to diagnose or treat the patient in need thereof; preferably, the tumors include solid tumors and hematologic malignancies; more preferably, the tumors include B-cell NHL, chronic lymphocytic leukemia (CLL), hairy cell leukemia (HCL), Hodgkin's disease, multiple myeloma, bladder cancer, kidney cancer, ovarian cancer, cervical cancer, breast cancer, lung cancer, nasopharyngeal cancer, malignant melanoma, pancreatic cancer and colon cancer.

FIG. 1A

FIG. 1B

FIG. 1C

FIG. 1D

FIG. 2A

FIG. 2B

FIG. 2C

FIG. 2D

FIG. 2E

FIG. 3A

FIG. 3B

FIG. 3C

FIG. 3D

FIG. 4A

FIG. 4B

FIG. 4C

FIG. 4D

FIG. 5A

FIG. 5B

FIG. 6A

FIG. 6B

FIG. 6C

FIG. 6D

FIG. 7

FIG. 8A

FIG. 8B

## INTERNATIONAL SEARCH REPORT

| International application No. |
| --- |
| **PCT/CN2023/085538** |

**A. CLASSIFICATION OF SUBJECT MATTER**

C07K16/28(2006.01)i;A61K39/395(2006.01)i;A61K39/00(2006.01)i;A61P35/00(2006.01)i

According to International Patent Classification (IPC) or to both national classification and IPC

**B. FIELDS SEARCHED**

Minimum documentation searched (classification system followed by classification symbols)

IPC: C07K16, A61K39, A61P35, C12N15

Documentation searched other than minimum documentation to the extent that such documents are included in the fields searched

Electronic data base consulted during the international search (name of data base and, where practicable, search terms used)

CNTXT, ENTXT, ENTXTC, OETXT, VEN, CJFD, CNKI, 万方, WANFANG, PubMed, ISI web of knowledge, 中国专利生物序列检索系统, China Patent Biological Sequence Search System, Genbank, EMBL-EBI, STN: CD40, TNFRSF5, 抗体, 肿瘤, 癌症, antibody, tumo?r, cancer, SEQ ID NO: 7, 15, 23, 33, 41, 49-73, 81, 85-109

**C. DOCUMENTS CONSIDERED TO BE RELEVANT**

| Category* | Citation of document, with indication, where appropriate, of the relevant passages | Relevant to claim No. |
| --- | --- | --- |
| A | CN 109071665 A (CELLDEX THERAPEUTICS INC.) 21 December 2018 (2018-12-21) <br> claims 1-55, description embodiments 1-21 | 1-17 |
| A | CN 113164781 A (BRISTOL-MYERS SQUIBB COMPANY) 23 July 2021 (2021-07-23) <br> claims 1-35, description embodiments 1-5 | 1-17 |
| A | WO 2018219327 A1 (JIANGSU HENGRUI MEDICINE CO., LTD. et al.) 06 December 2018 <br> (2018-12-06) <br> claims 1-26, description embodiments 1-11 | 1-17 |
| A | WO 2020154335 A1 (REVMAB BIOSCIENCES USA, INC.) 30 July 2020 (2020-07-30) <br> description embodiments 1-8, claims 1-29 | 1-17 |
| A | 张界等 (ZHANG, Jie et al.). "CD40单克隆抗体的制备、筛选及功能鉴定 (The Screen and Functional Study of CD40 Monoclonal Antibody)" <br> 现代免疫学 (Current Immunology), Vol. 37, No. (5), 31 May 2017 (2017-05-31), pp. 353-359 <br> ISSN: 1001-2478, <br> abstract | 1-17 |

☑ Further documents are listed in the continuation of Box C. ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **10 May 2023** | **02 June 2023** |

| Name and mailing address of the ISA/CN | Authorized officer |
| --- | --- |
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/085538**

### C. DOCUMENTS CONSIDERED TO BE RELEVANT

| Category* | Citation of document, with indication, where appropriate, of the relevant passages | Relevant to claim No. |
|---|---|---|
| A | Turner, J.G. et al. "Anti-CD40 Antibody Induces Antitumor and Antimetastatic Effects: the Role of NK Cells" *J Immunol*, Vol. 166, No. (1), 01 January 2001 (2001-01-01), pp. 89-94 ISSN: 0022-1767, abstract | 1-17 |

Form PCT/ISA/210 (second sheet) (July 2022)

**INTERNATIONAL SEARCH REPORT**

International application No.

**PCT/CN2023/085538**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

a.  ☑  forming part of the international application as filed.

b.  ☐  furnished subsequent to the international filing date for the purposes of international search (Rule 13*ter*.1(a)),

☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**

International application No.

**PCT/CN2023/085538**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **18**
   because they relate to subject matter not required to be searched by this Authority, namely:

   Claim 18 sets forth a method for diagnosing, treating and/or preventing tumors, which can be used for the diagnosis or treatment of diseases of the human or animal body, and therefore does not comply with PCT Rule 39.1(iv) and belongs to subject matter to be excluded.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2023/085538**

| Patent document cited in search report | | | Publication date (day/month/year) | Patent family member(s) | | | Publication date (day/month/year) |
|---|---|---|---|---|---|---|---|
| CN | 109071665 | A | 21 December 2018 | CA | 3021328 | A1 | 26 October 2017 |
| | | | | WO | 2017184619 | A2 | 26 October 2017 |
| | | | | AU | 2017252527 | A1 | 08 November 2018 |
| | | | | KR | 20180133493 | A | 14 December 2018 |
| | | | | KR | 102414558 | B1 | 29 June 2022 |
| | | | | JP | 2022088417 | A | 14 June 2022 |
| | | | | US | 2020369768 | A1 | 26 November 2020 |
| | | | | US | 10941201 | B2 | 09 March 2021 |
| | | | | IL | 262269 | A | 29 November 2018 |
| | | | | JP | 7038064 | B2 | 17 March 2022 |
| | | | | EA | 201892362 | A1 | 30 April 2019 |
| | | | | US | 2021147538 | A1 | 20 May 2021 |
| | | | | EP | 3445783 | A2 | 27 February 2019 |
| | | | | SG | 11201808821 | WA | 29 November 2018 |
| | | | | MA | 44723 | A | 27 February 2019 |
| | | | | US | 2019322743 | A1 | 24 October 2019 |
| | | | | US | 10633444 | B2 | 28 April 2020 |
| | | | | US | 2020377606 | A1 | 03 December 2020 |
| | | | | BR | 112018071307 | A2 | 26 February 2019 |
| | | | | US | 2018066053 | A1 | 08 March 2018 |
| | | | | US | 10865244 | B2 | 15 December 2020 |
| | | | | SG | 10201913248 | A1 | 27 February 2020 |
| | | | | US | 2021147538 | A1 | 20 May 2021 |
| | | | | CN | 109071665 | B | 01 November 2022 |
| | | | | MX | 2018012757 | A1 | 24 June 2019 |
| | | | | IN | 201817042470 | A | 04 January 2019 |
| CN | 113164781 | A | 23 July 2021 | BR | 112021009140 | A2 | 17 August 2021 |
| | | | | EA | 202191359 | A1 | 21 October 2021 |
| | | | | US | 2020157233 | A1 | 21 May 2020 |
| | | | | US | 11261258 | B2 | 01 March 2022 |
| | | | | EP | 3883652 | A1 | 29 September 2021 |
| | | | | TW | 202033556 | A | 16 September 2020 |
| | | | | US | 2022153856 | A1 | 19 May 2022 |
| | | | | CO | 2021007691 | A2 | 20 September 2021 |
| | | | | CL | 2021001230 | A1 | 07 January 2022 |
| | | | | AU | 2019385331 | A1 | 01 July 2021 |
| | | | | IL | 283104 | A | 30 June 2021 |
| | | | | US | 2021054090 | A1 | 25 February 2021 |
| | | | | US | 11254750 | B2 | 22 February 2022 |
| | | | | WO | 2020106620 | A1 | 28 May 2020 |
| | | | | KR | 20210093968 | A | 28 July 2021 |
| | | | | PE | 20211293 | A1 | 20 July 2021 |
| | | | | US | 2022169742 | A1 | 02 June 2022 |
| | | | | CA | 3120358 | A1 | 28 May 2020 |
| | | | | US | 2022144962 | A1 | 12 May 2022 |
| | | | | AR | 117091 | A1 | 07 July 2021 |
| | | | | IN | 202117026423 | A | 19 November 2021 |
| | | | | ZA | 202103353 | A | 21 December 2022 |
| | | | | CN | 113164781 | B | 28 February 2023 |
| | | | | SG | 11202104776 | A1 | 29 June 2021 |

Form PCT/ISA/210 (patent family annex) (July 2022)

INTERNATIONAL SEARCH REPORT
Information on patent family members

International application No.

**PCT/CN2023/085538**

| Patent document cited in search report | | | Publication date (day/month/year) | Patent family member(s) | | | Publication date (day/month/year) |
|---|---|---|---|---|---|---|---|
| WO | 2018219327 | A1 | 06 December 2018 | JP | 2020521504 | A | 27 July 2020 |
| | | | | JP | 7257971 | B2 | 14 April 2023 |
| | | | | CA | 3064298 | A1 | 06 December 2018 |
| | | | | US | 2020148778 | A1 | 14 May 2020 |
| | | | | US | 11525005 | B2 | 13 December 2022 |
| | | | | KR | 20200012920 | A | 05 February 2020 |
| | | | | BR | 112019025048 | A2 | 30 June 2020 |
| | | | | AU | 2018278051 | A1 | 30 January 2020 |
| | | | | RU | 2019141751 | A | 12 July 2021 |
| | | | | EP | 3632932 | A1 | 08 April 2020 |
| | | | | TW | 201902925 | A | 16 January 2019 |
| | | | | CN | 110267989 | A | 20 September 2019 |
| | | | | MX | 2019014375 | A1 | 23 January 2020 |
| | | | | TW | 699376 | B1 | 21 July 2020 |
| | | | | RU | 2779128 | C2 | 02 September 2022 |
| WO | 2020154335 | A1 | 30 July 2020 | US | 2022089758 | A1 | 24 March 2022 |
| | | | | CN | 113677365 | A | 19 November 2021 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2022103511889

**Non-patent literature cited in the description**

- **KABAT et al.** Sequences of Proteins of Immunological Interest. National Institutes of Health, 1991

- *JMol Biol*, 1997, vol. 273, 927-48